# EUROPEAN PATENT APPLICATION

(11) **EP 4 124 345 A1**
(43) Date of publication of application: **01.02.2023**
(21) Application number: 21382723.1
(22) Date of filing: 30.07.2021
(51) Int. Cl.: A61K 47/69

(54) **NANOPARTICLES FOR MUSCLE DELIVERY**

(71) Applicant: 4basebio UK Ltd, Cambridge CB24 5QE (GB); 4basebio, S.L.U., 28049 Cantoblanco (Madrid) (ES)
(72) Inventor: Walker, Amy, Over, Cambridge, CB24 5QE (GB); Lanckriet, Heikki, Over, Cambridge, CB24 5QE (GB); Picher, Angel, 28049 Cantoblanco (Madrid) (ES); Dyankova, Elena, Over, Cambridge, CB24 5QE (GB)
(74) Representative: Boult Wade Tennant LLP

(57) **Abstract**

Nanoparticles suitable for delivery of a cargo to a muscle cell, and targeting peptides comprising a muscle cell targeting sequence, are provided. Further provided are uses of the nanoparticles and targeting peptides, for example, in treating a muscle disease or disorder.

## Description

### TECHNICAL FIELD

The present invention relates to nanoparticles suitable for delivery of a cargo to a muscle cell. In addition, the present invention relates to targeting peptides comprising a muscle cell targeting sequence. The present invention also relates to uses of the nanoparticles and targeting peptides, for example, in treating a muscle disease.

### BACKGROUND

Muscular dystrophies are a group of inherited diseases that cause progressive weakness and loss of muscle mass, leading to reduced motor function and coordination. There are over thirty disorders classed as muscular dystrophies, including Duchenne's muscular dystrophy (DMD), Beker muscular dystrophy and myotonic dystrophy. DMD is an X-linked inherited disease caused by mutations in the DMD gene, with an incidence rate of 1 in 3,500-6000 live male births. The DMD gene encodes dystrophin, an integral cytoplasmic protein that forms part of a protein complex that connects the cytoskeleton of a muscle fibre to the surrounding extracellular matrix through the cell membrane. Dystrophin plays a critical role in stabilising the sarcolemma during muscle contraction and, in its absence, muscle integrity and contractility is compromised. This contractile damage, in the short term, promotes muscle regeneration, but continued cycles of contractile damage and regeneration can lead to fibrosis, propagating muscle wasting, scarring and fat replacement in DMD. Clinical manifestations of the disease initially develop in the proximal skeletal muscles, with most children becoming wheelchair bound between the ages of 10-12 years old. Manifestations progress to the respiratory and cardiac systems, ultimately causing death.

Despite major therapeutic advances over the past 30 years, there is no cure for DMD. As a monogenic disease, DMD has long been regarded as amenable to a gene therapy strategy. Owing to the large length of the dystrophin gene and limited packaging capacity of vectors such as an adeno-associated virus (AAV) vector, the vast majority of therapeutic approaches have focused on the delivery of a highly abbreviated, yet partially functional micro-dystrophin gene, which can produce a Becker's muscular dystrophy phenotype with less clinical burden. Several AAV vectors delivering a form of micro-dystrophin are in early-stage clinical trials, however key domains of the dystrophin gene have been removed, which may lead to sub-optimal expression.

More generally although viruses as delivery agents are considered to have the advantages of high efficiency and high cell selectivity, they have several disadvantages including toxicity, risk of insertional mutagenesis, production of inflammatory responses, high likelihood of eliciting a host immune response and limited packaging capacity.

Non-viral vectors such as lipid-based nanoparticles provide a method of targeted delivery and controlled release of therapeutic agents such as drugs and nucleic acids to cells. Lipid-based nanoparticles (particularly nanoparticles comprising cationic lipids) have been investigated as a non-viral vector for gene delivery.

Liposomes have a spherical lipid bilayer, mostly comprised of phospholipids with a hydrophilic head group and hydrophobic tail. The positively-charged head groups of cationic lipids can facilitate spontaneous electrostatic binding with negatively charged phosphate groups on DNA molecules, forming entropically favourable nanoparticles. The net charge of the nanoparticles is determined by the ratio between the free amine groups and the phosphate groups in the nanoparticle, which, in turn, affects the size, stability, and structure of the particles.

Felgner et al. ("Lipofection: a highly efficient, lipid-mediated DNA-transfection procedure" Proceedings of the National Academy of Sciences 84.21 (1987): 7413-7417) describes the application of cationic lipids as gene vectors. In addition, there are now many commercially available, widely used lipids designed and synthesised specifically for transfection, with different structural aspects such as head group and hydrocarbon tail length. A neutral, 'helper' lipid such as DOPE (Dioleoyl L-α phosphatidyl ethanolamine) is now often formulated with cationic lipids because of its membrane stabilising properties, which are believed to aid endosomal escape. Cholesterol, an important component of biological cell membranes, can be added to increase circulation time of lipid nanoparticles by imparting stability to the complexes. Meanwhile, PEGylation of liposomal particles, (with poly(ethylene glycol)), can prevent aggregation of nanoparticles, with particles remaining small and discrete both outside and within cells, whilst conveying a stealth coating on the particles, enhancing serum stability.

WO 02/072616 A2 describes an exemplary non-viral transfection complex, which comprises: (a) a nucleic acid, (b) a lipid component, (c) a polycationic nucleic acid-binding component, and (d) a cell surface receptor binding component comprising a peptide that binds to human airway epithelial cells.

A need exists for an improved delivery system that does not suffer from the disadvantages of viral vectors and which allows for specific targeting of muscle cells.

### DESCRIPTION

The invention provides a nanoparticle suitable for delivery of a cargo to a target cell. The invention provides a nanoparticle comprising (a) a cargo, (b) a lipid component, and (c) a targeting peptide. The nanoparticle is preferably a non-viral delivery system, such as a non-viral transfection complex. The invention preferably targets muscle cells. The cargo may be a nucleic acid. The nucleic acid may have an enhanced resistance to nuclease (e.g. exonuclease) digestion. The cargo may comprise a cassette. The cassette may comprise a coding sequence. The coding sequence may encode a gene useful in treating a disease or disorder (e.g. a muscle disease or disorder).

The present inventors have developed a nanoparticle (e.g. a non-viral transfection complex) suitable for use in treating a muscle disease or disorder. The nanoparticle of the present invention has several advantages over viral delivery systems. Firstly, the nanoparticle of the present invention is less likely to elicit an immune response, which is particularly important as repeated doses may need to be administered. In addition, the nanoparticle of the present invention can be used to deliver much larger cargos to a cell (e.g. muscle cell).

The nanoparticles of the present invention provide further benefits in that they are cost-effective and simple to produce on a large scale.

The nanoparticles of the present invention may comprise a nucleic acid cargo (e.g. linear DNA) that has an enhanced resistance to nuclease (e.g. exonuclease) digestion, which results in a prolonged in vivo life of the cargo molecule.

Importantly, the nanoparticles of the present invention provide improved targeting to muscle cells as compared to any other non-viral delivery systems.

### 1. Nanoparticles and non-viral transfection complexes

The invention provides a nanoparticle suitable for delivery of a cargo to a target cell. The nanoparticle comprises: (a) a cargo; (b) a lipid component; and (c) a targeting peptide. The targeting peptide may improve targeting of a nanoparticle to a desired location, for example, a muscle cell.

The invention provides, a nanoparticle comprising:
(a) a cargo;
(b) a lipid component; and
(c) a targeting peptide comprising a muscle cell targeting sequence.

The term "muscle cell targeting sequence" refers to a sequence that has the ability to target, bind and/or interact with a muscle cell. For example, the muscle cell targeting sequence may target, bind and/or interact with a receptor on a muscle cell, or a biologically active molecule present on the surface of the muscle cell.

The muscle cell targeting sequence may be a muscle-specific targeting sequence. The term "specific" refers to the ability to preferentially target, bind and/or interact with a given target, for example, a receptor on a muscle cell, or a biologically active molecule present on the surface of the muscle cell, over other targets. Preferential targeting, binding and/or interacting with may be assessed by measuring the binding affinity of a sequence for a target on a muscle cell in comparison to a target on a different cell. Similarly, preferential targeting, binding and/or interacting with may be measured by calculating a dissociation rate (of a sequence from a target molecule). Preferential targeting may be assessed by comparing the amount of binding events for each one of the target cells. For example, the muscle-specific targeting sequence may bind to a muscle cell more often than any other cell type. The muscle-specific targeting sequence may bind with higher affinity for a muscle cell (or a receptor on a muscle cell, or a biologically active molecule present on the surface of the muscle cell) than any other cell (or receptor) type.

The nanoparticle may comprise:
(a) a cargo;
(b) a lipid component; and
(c) a targeting peptide comprising a muscle-specific targeting sequence.

The nanoparticle may be for delivery of a cargo to a muscle cell. Preferably, the nanoparticle is for the cell-specific delivery of a cargo to a muscle cell.

The nanoparticle may be for delivery of any type of a cargo to a target cell. Preferably, the cargo has beneficial (e.g. therapeutic) effect on the target cell. The cargo may be a biomolecule. The cargo may be a small molecule. The cargo may be branched, linear, or spaced. The biomolecule may be a nucleic acid, a peptide, a polypeptide, or a protein. The biomolecule may be a fragment of a nucleic acid, a fragment of a peptide, a fragment of polypeptide, or a fragment of a protein.
The nucleic acid may be a DNA molecule or an RNA molecule. The DNA molecule may be a linear DNA molecule or circular DNA molecule. The nucleic acid may be single-stranded, double-stranded, or partially single-stranded and partially double-stranded. The nucleic acid may comprise one or more protected nucleotides (i.e. nucleotides resistant to nuclease (e.g. exonuclease) digestion) (e.g. phosphorothioated nucleotides).

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid comprises at least 500 nucleotides.

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid comprises at least 500 base pairs.

The circular DNA molecule may be a plasmid DNA, a vector DNA, a cosmid, an isolated DNA, a bacterial artificial chromosome, a minicircle, or a mini intronic plasmid (MIP). The circular DNA may be an enzymatically produced circular DNA molecule. For example, (i) a circular DNA molecule obtained from recombinase reaction (e.g. Cre recombinase reaction) or (ii) a circular DNA molecule obtained from ligase reaction (e.g. using the golden gate assembly).

The linear DNA molecule may be a chromosome. The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides).

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The RNA molecule may be a messenger RNA (mRNA), a transfer RNA, a ribosomal RNA, a small interfering RNA (siRNA), an antisense RNA (an antisense oligonucleotide), a small nuclear RNA (snRNA), a double-stranded RNA, a microRNA (miRNA), short hairpin RNA (shRNA), guide RNA (gRNA), self-amplifying RNA (samRNA), or circular RNA.

The RNA molecule may comprise at least 5, at least 10, at least 15, at least 20, at least 25, at least 30, at least 35, at least 40, at least 45, at least 50, at least 75, at least 100, at least 150, or at least 200 nucleotides. The RNA molecule may comprise 10-13,000, 15-10,000, 20-5,000, 20-1,000, 20-500, or 25-300 nucleotides. The RNA molecule may comprise one or more protected nucleotides (i.e. nucleotides resistant to nuclease (e.g. exonuclease) digestion) (e.g. phosphorothioated nucleotides).

The siRNA may comprise a double-stranded portion of at least 17 base pairs, at least 18 base pairs or preferably at least 19 base pairs. The siRNA may comprise a double stranded portion of 17-30 base pairs, 18-27 base pairs, 19-24 base pairs or preferably 19-21 base pairs. The siRNA may comprise one or more protected nucleotides (i.e. nucleotides resistant to nuclease (e.g. exonuclease) digestion) (e.g. phosphorothioated nucleotides).

The antisense RNA may comprise at least 18, at least 19, at least 20, at least 21, at least 22, or at least 23 nucleotides. The antisense RNA may comprise 18-24 nucleotides or preferably 19-23 nucleotides. The antisense RNA may comprise one or more protected nucleotides (i.e. nucleotides resistant to nuclease (e.g. exonuclease) digestion) (e.g. phosphorothioated nucleotides).

The protein may be a protein of a CRISPR system, for example, Cas9, Cas13, or Cpf1. The protein may be a therapeutic protein. That is to say that the therapeutic protein, once delivered to a target cell, elicits a therapeutic effect. The protein may be a base editing protein, or a prime editing protein.

The protein may be of at least 100 Da, at least 200 Da, at least 300 at least, at least 400 Da, at least 500 Da, at least 750 Da, at least 1 kDa, at least 5 kDa, at least 10 kDa, at least 25 kDa, at least 50 kDa, at least 75 kDa, at least 100 kDa, at least 125 kDa, at least 150 kDa, at least 160 kDa, or at least 175 kDa.

The small molecule may be an agonist (e.g. a receptor agonist), an antagonist (e.g. a receptor antagonist), an activator, or an inhibitor. The small molecule may be a chemical compound. The small molecule may be a drug of a chemical structure comprising 20-100 atoms. The small molecule may be a chemical compound of a molecular mass of less than 1 kDa. For example, the small molecule may be a PTEN inhibitor.

The nanoparticle may be a non-viral transfection complex. Thus, the invention provides a non-viral transfection complex comprising:
(a) a cargo
(b) a lipid component; and
(c) a targeting peptide comprising a muscle cell targeting sequence.

Preferably, the cargo in the non-viral transfection complex is a nucleic acid. Thus, the non-viral transfection complex may comprise:
(a) a cargo, wherein the cargo is a nucleic acid;
(b) a lipid component; and
(c) a targeting peptide comprising a muscle cell targeting sequence.

The nanoparticle (e.g. the non-viral transfection complex) may further comprise a cationic component, an anionic component, and/or a neutral component. The nanoparticle may comprise a cargo-binding component. The cargo-binding component may be a cargo-binding cationic component, a cargo-binding neutral component or a cargo-binding anionic component. The cationic component, the anionic component, and/or the neutral component may be used to establish a desired charge (i.e. a negative/positive ratio) of the nanoparticle. A specific charge (i.e. a Nitrogen/Phosphate ratio) may be required to facilitate or enhance cell transfection. Thus, the nanoparticle (or a non-viral transfection complex) may comprise:
(a) a cargo
(b) a lipid component;
(c) a targeting peptide comprising a muscle cell targeting sequence; and
(d) a peptide comprising a cargo-binding component.

The cargo-binding component may be a biomolecule-binding component. The biomolecule-binding component may be a biomolecule-binding cationic component, a biomolecule-binding neutral component or a biomolecule-binding anionic component. The cargo-binding component may be a small molecule-binding component. The small molecule-binding component may be a small molecule-binding cationic component, a small molecule-binding neutral component or a small molecule-binding anionic component.

The cargo-binding component may be a nucleic acid-binding component. The nucleic acid-binding component may be a nucleic acid-binding cationic component or a nucleic acid-binding neutral component.

The nucleic acid-binding component may be a DNA-binding component. The DNA-binding component may be a DNA-binding cationic component or a DNA-binding neutral component. The nucleic acid-binding component may be a closed linear DNA-binding component. The closed linear DNA-binding component may be a closed linear DNA-binding cationic component or a closed linear DNA-binding neutral component. The nucleic acid-binding component may be a linear DNA-binding component. The linear DNA binding component may be a linear DNA-binding cationic component, or a linear DNA-binding neutral component. The nucleic acid-binding component may be an RNA-binding component. The RNA-binding component may be an RNA-binding cationic component, or an RNA-binding neutral component. The nucleic acid-binding component may be an antisense RNA-binding component. The antisense RNA-binding component may be an antisense RNA-binding cationic component, or an antisense RNA-binding neutral component. The nucleic acid-binding component may be a siRNA-binding component. The siRNA-binding component may be a siRNA-binding cationic component, or a siRNA-binding neutral component. The nucleic acid-binding component may be an mRNA-binding component. The mRNA-binding component may be an mRNA-binding cationic component, or an mRNA-binding neutral component. The nucleic acid-binding component may be a transfer RNA-binding component. The transfer RNA-binding component may be a transfer RNA-binding cationic component, or a transfer RNA-binding neutral component. The nucleic acid-binding component may be a ribosomal RNA-binding component. The ribosomal RNA-binding component may be a ribosomal RNA-binding cationic component, or a ribosomal RNA-binding neutral component. The nucleic acid-binding component may be an snRNA-binding component. The snRNA-binding component may be an snRNA-binding cationic component, or an snRNA-binding neutral component. The nucleic acid-binding component may be a double-stranded RNA-binding component. The double-stranded RNA-binding component may be a double-stranded RNA-binding cationic component, or a double-stranded RNA-binding neutral component. The nucleic acid-binding component may be an miRNA-binding component. The miRNA-binding component may be an miRNA-binding cationic component, or an miRNA-binding neutral component. The nucleic acid-binding component may be a shRNA-binding component. The shRNA-binding component may be a shRNA-binding cationic component, or a shRNA-binding neutral component. The nucleic acid-binding component may be a gRNA-binding component. The gRNA-binding component may be a gRNA-binding cationic component, or a gRNA-binding neutral component. The nucleic acid-binding component may be a samRNA-binding component. The samRNA-binding component may be a samRNA-binding cationic component, or a samRNA-binding neutral component. The nucleic acid-binding component may be a circular RNA-binding component. The circular RNA-binding component may be a circular RNA-binding cationic component, or a circular RNA-binding neutral component.

The cargo-binding component may be a protein-binding component. The protein-binding component may be a protein-binding cationic component, a protein-binding neutral component or a protein-binding anionic component. The protein-binding component may be a Cas9-binding component or Cpf1-binding component. The Cas9 binding component may be a Cas9-binding cationic component, a Cas9-binding neutral component or a Cas9-binding anionic component. The Cpf1 binding component may be a Cpf1-binding cationic component, a Cpf1-binding neutral component or a Cpf1-binding anionic component.

The cargo-binding component may be a peptide-binding component. The peptide-binding component may be a peptide-binding cationic component, a peptide-binding neutral component or a peptide-binding anionic component.

The cargo-binding component may be a polypeptide-binding component. The polypeptide-binding component may be a polypeptide-binding cationic component, a polypeptide-binding neutral component or a polypeptide-binding anionic component.

If the cargo is a nucleic acid, the nanoparticle (e.g. the non-viral transfection complex) may comprise a nucleic acid-binding cationic component or a nucleic acid-binding neutral component. Thus, the nanoparticle (or a non-viral transfection complex) may comprise:
(a) a cargo, wherein the cargo is a nucleic acid;
(b) a lipid component;
(c) a targeting peptide comprising a muscle cell targeting sequence; and
(d) a peptide comprising a nucleic acid-binding component.

Preferably, the nucleic acid-binding component is a nucleic acid-binding cationic component (e.g. DNA-binding cationic component).

The cargo-binding cationic component may be a cargo-binding polycationic component, The cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the cargo-binding polycationic component comprises at least 16, at least 17 or at least 30 cationic monomers.

The cargo-binding cationic component may comprise a lysine, a histidine, or an arginine. The cargo-binding polycationic component may comprise a lysine, a histidine, or an arginine. The cargo-binding polycationic component may comprise an oligolysine (linear or branched), an oligohistidine (linear or branched) or an oligoarginine (linear or branched). For example, the cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the cargo-binding polycationic component comprises at least 16, at least 17, or at least 30 lysine residues. More preferably still, the cargo-binding polycationic component comprises at least 17 lysine residues. The cargo-binding polycationic component may be linear or branched. For example, the cargo-binding linear polycationic component may comprise at least 17 lysine residues. The cargo-binding branched polycationic component may comprise at least 17 lysine residues.

The nanoparticle (or a non-viral transfection complex) may comprise:
(a) a cargo, wherein the cargo is a nucleic acid;
(b) a lipid component;
(c) a targeting peptide comprising a muscle cell targeting sequence; and
(d) a peptide comprising a nucleic acid-binding polycationic component,
wherein the nucleic acid-binding polycationic component is oligolysine comprising at least 17 lysine resides.

The nanoparticle (or a non-viral transfection complex) may comprise:
(a) a cargo, wherein the cargo is a nucleic acid;
(b) a lipid component;
(c) a targeting peptide comprising a muscle cell targeting sequence; and
(d) a peptide comprising a nucleic acid-binding polycationic component,
wherein the nucleic acid-binding polycationic component is oligolysine comprising at least 30 lysine resides.

The nanoparticle (or a non-viral transfection complex) may comprise:
(a) a cargo, wherein the cargo is a nucleic acid;
(b) a lipid component;
(c) a targeting peptide comprising a muscle cell targeting sequence; and
(d) a peptide comprising a nucleic acid-binding polycationic component,
wherein the nucleic acid-binding linear polycationic component is oligolysine comprising at least 17 lysine resides.

The nanoparticle (or a non-viral transfection complex) may comprise:
(a) a cargo, wherein the cargo is a nucleic acid;
(b) a lipid component;
(c) a targeting peptide comprising a muscle cell targeting sequence; and
(d) a peptide comprising a nucleic acid-binding polycationic component,
wherein the nucleic acid-binding branched polycationic component is oligolysine comprising at least 30 lysine resides.

The cargo-binding anionic component maybe be a cargo-binding polyanionic component, The cargo-binding polyanionic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 anionic monomers. The cargo-binding polyanionic component may be linear or branched. For example, the cargo-binding linear polyanionic component may comprise at least 17 monomers. The cargo-binding branched polyanionic component may comprise at least 17 monomers.

The cargo-binding neutral component maybe be a cargo-binding polyneutral component, The cargo-binding polyneutral component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 neutral monomers. The cargo-binding polyneutral component may be linear or branched. For example, the cargo-binding linear polyneutral component may comprise at least 17 monomers. The cargo-binding branched polyneutral component may comprise at least 17 monomers.

The nucleic acid-binding cationic component (e.g. the DNA-binding cationic component) may be a nucleic acid-binding polycationic component (e.g. a DNA-binding polycationic component). The nucleic acid-binding polycationic component may comprise a lysine. The nucleic acid-binding polycationic component may comprise an oligolysine. The nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the nucleic acid-binding polycationic component comprises at least 16, at least 17, or at least 30 lysine residues. More preferably still, the cargo-binding polycationic component comprises at least 17 lysine residues. The nucleic acid-binding polycationic component may be linear or branched. For example, the nucleic acid-binding linear polycationic component may comprise at least 17 lysine residues. The nucleic acid-binding branched polycationic component may comprise at least 17 lysine residues.

Thus, the nanoparticle (or a non-viral transfection complex) may comprise:
(a) a cargo, wherein the cargo is a nucleic acid;
(b) a lipid component;
(c) a targeting peptide comprising a muscle cell targeting sequence; and
(d) a peptide comprising a nucleic acid-binding polycationic component (e.g. DNA-binding polycationic component).

The cargo-binding component may be located on the same sequence at the targeting sequence (e.g. muscle cell targeting sequence). The cargo-binding component may be a part of the targeting peptide. Thus, the invention provides a nanoparticle (or a non-viral transfection complex) comprising:
(a) a cargo
(b) a lipid component; and
(c) a targeting peptide comprising a muscle cell targeting sequence and a cargo-binding component.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding component (e.g. a DNA-binding polycationic component).

Preferably, the nanoparticle (e.g. the non-viral transfection complex) comprises:
(a) a cargo, wherein the cargo is a nucleic acid;
(b) a lipid component; and
(c) a targeting peptide comprising a muscle cell targeting sequence and a nucleic acid-binding cationic component (e.g. a DNA-binding polycationic component).

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding polycationic component (e.g. a DNA-binding polycationic component).

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding polycationic component (e.g. a DNA-binding polycationic component),
wherein the nucleic acid-binding polycationic component comprises oligolysine, optionally wherein the oligolysine comprises at least 17 lysine residues.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding polycationic component (e.g. a DNA-binding polycationic component),
wherein the nucleic acid-binding polycationic component comprises oligolysine, optionally wherein the oligolysine comprises at least 30 lysine residues.

The cargo may be a linear DNA molecule with enhanced resistance to nuclease (e.g. exonuclease digestion). Thus, the nanoparticle (e.g. the non-viral transfection complex) may comprise:
(a) a cargo, wherein the cargo is a nucleic acid, wherein the nucleic acid is a closed linear DNA molecule;
(b) a lipid component; and
(c) a targeting peptide comprising a muscle cell targeting sequence and a closed linear DNA-binding component (e.g. a closed linear DNA-binding polycationic component).

The cargo may be a linear DNA molecule with enhanced resistance to nuclease (e.g. exonuclease digestion). Thus, the nanoparticle (e.g. the non-viral transfection complex) may comprise:
(a) a cargo, wherein the cargo is a nucleic acid, wherein the nucleic acid is a closed linear DNA molecule;
(b) a lipid component; and
(c) a targeting peptide comprising a muscle cell targeting sequence and a closed linear DNA-binding component (e.g. a closed linear DNA-binding polycationic component);
wherein the closed linear DNA-binding component comprises oligolysine, optionally wherein the oligolysine comprises at least 17 lysine residues.

The cargo may be a linear DNA molecule with enhanced resistance to nuclease (e.g. exonuclease digestion). Thus, the nanoparticle (e.g. the non-viral transfection complex) may comprise:
(a) a cargo, wherein the cargo is a nucleic acid, wherein the nucleic acid is a closed linear DNA molecule;
(b) a lipid component; and
(c) a targeting peptide comprising a muscle cell targeting sequence and a closed linear DNA-binding component (e.g. a closed linear DNA-binding polycationic component);
wherein the closed linear DNA-binding component comprises oligolysine, optionally wherein the oligolysine comprises at least 30 lysine residues.

The nanoparticle (e.g. the non-viral transfection complex) may comprise:
(a) a cargo, wherein the cargo is a nucleic acid, wherein the nucleic acid is a linear DNA molecule comprising one or more nuclease-resistant nucleotides (e.g. phosphorothioated nucleotides);
(b) a lipid component; and
(c) a targeting peptide comprising a muscle cell targeting sequence and a linear DNA-binding component (e.g. a linear DNA-binding polycationic component).

The nanoparticle (e.g. the non-viral transfection complex) may comprise:
(a) a cargo, wherein the cargo is a nucleic acid, wherein the nucleic acid is a linear DNA molecule comprising one or more nuclease-resistant nucleotides (e.g. phosphorothioated nucleotides);
(b) a lipid component; and
(c) a targeting peptide comprising a muscle cell targeting sequence and a linear DNA-binding component (e.g. a linear DNA-binding polycationic component),
wherein the linear DNA-binding component comprises oligolysine, optionally wherein the oligolysine comprises at least 17 lysine residues.

The nanoparticle (e.g. the non-viral transfection complex) may comprise:
(a) a cargo, wherein the cargo is a nucleic acid, wherein the nucleic acid is a linear DNA molecule comprising one or more nuclease-resistant nucleotides (e.g. phosphorothioated nucleotides);
(b) a lipid component; and
(c) a targeting peptide comprising a muscle cell targeting sequence and a linear DNA-binding component (e.g. a linear DNA-binding polycationic component),
wherein the linear DNA-binding component comprises oligolysine, optionally wherein the oligolysine comprises at least 30 lysine residues.

The cargo-binding component may be linear or branched. The cargo-binding polycationic component may be linear or branched. The cargo-binding polyanionic component may be linear or branched. The cargo-binding polyneutral component may be linear or branched. For example, the cargo-binding polycationic component may comprise at least 16, at least 17, or at least 30 lysine residues in a linear chain. Alternatively, the cargo-binding polycationic component may comprise at least 16, at least 17, or at least 30 lysine residues in a branched chain. The cargo may be a nucleic acid-binding polycationic component. The nucleic acid-binding polycationic component may be linear or branched. For example, the nucleic acid-binding polycationic component (e.g. the DNA-binding polycationic component) may comprise at least 16, at least 17, or at least 30 lysine residues in a linear chain. Alternatively, the nucleic acid-binding polycationic component (e.g. the DNA-binding polycationic component) may comprise at least 16, at least 17, or at least 30 lysine residues in a branched chain.

The lipid component may be or may form a liposome. The lipid component may comprise a cationic lipid, an anionic lipid, or a neutral lipid. The lipid component may be or may form an anionic liposome, a cationic liposome, or a neutral liposome.

The liposome may comprise at least one lipid. The liposome may comprise at least one cationic lipid. The liposome may comprise at least one phospholipid. The liposome may comprise at least one cationic lipid and at least one phospholipid. The liposome may comprise at least one steroid lipid. The liposome may comprise at least one cationic lipid and at least one steroid lipid. The liposome may comprise at least one phospholipid and at least one steroid lipid. The liposome may comprise at least one cationic lipid, at least one phospholipid and at least one steroid lipid. The liposome may comprise at least one ionizable lipid. The liposome may comprise at least one anionic lipid.

The cationic lipid may be DTDTMA (ditetradecyl trimethyl ammonium), DOTMA (2,3-dioleyloxypropy1-1-trimentyl ammonium), or DHDTMA (dihexadecyl trimethyl ammonium). In addition to the cation, the cationic lipids may comprise a counter anion, for example, an inorganic counter ion, especially a pharmaceutically acceptable anion such as chloride or bromide. Preferably, the cationic lipid is DOTMA.

The lipid component may comprise a phospholipid. The term "phospholipid" refers to a lipid comprising a fatty acid chin and a phosphate group. Phospholipids are typically neutral molecules in that they do not have an overall charge, unlike a cationic lipid, which is positively charged. Phospholipids are typically zwitterionic molecules comprising both positive and negative charged components, but no overall charge. Thus, the neutral lipid may be a phospholipid. For example, the phospholipid may be DOPE (phosphatidylethanolamine or 1,2-dioleoyl-sn-glycero-3-phosphoethanolamine), or DOPC (phosphatidyl choline or 1,2-dioleoyl- sn-glycero-3-phosphoethanoltrimethylamine). Preferably, the phospholipid is DOPE.

The phospholipid may comprise a PEG moiety. The PEG moiety may have a molecular weight of from about 100 to about 10,000, optionally the PEG moiety has a molecular weight of from about 250 to about 7,500, optionally the PEG moiety has a molecular weight of from about 500 to about 5,000, optionally the PEG moiety has a molecular weight of from about 750 to about 4,000, optionally the PEG moiety has a molecular weight of from about 1,000 to about 3,000, optionally the PEG moiety has a molecular weight of approximately 2,000.

The ionizable lipid may be (6Z,9Z,28Z,31Z)-heptatriaconta-6,9,28,31-tetraen-19-yl-4-(dimethylamino)-butanoate (Dlin-MC3-DMA (MC3)) or 2,2-dilinoleyl-4-(2- dimethylaminoethyl)-[1,3]-dioxolane (DLin-KC2-DMA (KC2))

The anionic lipid may be 1,2-Dioleoyl-sn-glycero-3-phosphoglycerol (DOPG).

The steroid lipid may be cholesterol. The liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, at least 25%, at least 30% at least 35%, at least 40%, at least 45%, at least 50%, or at least 55% cholesterol (as defined by molar amount of cholesterol). That is to say that the liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, or at least 25% cholesterol and at least 99%, at least 98%, at least 97%, at least 96% at least 95%, at least 94%, at least 93%, at least 92%, at least 91%, at least 90%, at least 89%, at least 88%, at least 87%, at least 86%, at least 85%, at least 84%, at least 83%, at least 82%, at least 81%, at least 80% at least 79%, at least 78%, at least 77%, at least 76%, at least 75%, at least 70%, at least 65%, at least 60%, at least 55%, at least 50%, or at least 45% of other lipids in the liposome (as defined by molar ratio).

The molar ratio of at least one cationic lipid to at least one phospholipid in the nanoparticle (or non-viral transfection complex) may be 1:1, 1:2, 1:3, 1:4, 1:5, 2:1, 3:1, 4:1, or 5:1. Preferably, the molar ratio of at least one cationic lipid to at least one phospholipid in the nanoparticle (or non-viral transfection complex) is 1:1 or 2:1. For example, the molar ratio of DOTMA to DOPE in the nanoparticle (or non-viral transfection complex) may be 1:1. That is to say that the molar amount of DOTMA and DOPE in the nanoparticle is the same. The molar ratio of DOTMA to DOPE in the nanoparticle (or non-viral transfection complex) may be 2:1. That is to say that the molar amount of DOTMA is twice the molar amount of DOPE.

The targeting sequence may be a muscle cell targeting sequence. The muscle cell targeting sequence may be a skeletal muscle cell targeting sequence, a cardiac muscle cell targeting sequence, or a smooth muscle cell targeting sequence. Preferably, the muscle cell targeting sequence is the skeletal muscle cell targeting sequence.

The muscle cell targeting sequence may preferentially bind to the skeletal muscle cell over the smooth muscle cell or the cardiac muscle cell. That is to say that the muscle cell targeting sequence is a skeletal muscle cell-specific targeting sequence. The muscle cell targeting sequence may preferentially bind to the cardiac muscle cell over the smooth muscle cell. That is to say that the muscle cell targeting sequence is a cardiac muscle cell-specific targeting sequence.

The targeting sequence may comprise at least 3, at least 4, at least 5, at least 6, at least 7, at least 8, at least 9, at least 10, at least 11, at least 12, at least 13 or at least 14 amino acids. The targeting sequence may comprise 3-30, 4-20, 5-17, 6-15, or 7-14 amino acids. Preferably, the targeting sequence comprises 7-14 amino acids. For example, the targeting sequence may comprise 7 or 12 amino acids.

The targeting sequence (e.g. the muscle cell targeting sequence) may comprise at least 3, at least 4, at least 5 or at least 6 contiguous amino acids of SEQ ID NO: 1 or a variant thereof comprising one or more conservative amino acid substitutions. The targeting sequence (e.g. the muscle cell targeting sequence) may be SEQ ID NO: 1 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:1. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:1.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 2 or a variant thereof comprising one or more conservative amino acid substitutions. The targeting sequence (e.g. the muscle cell targeting sequence) may be SEQ ID NO: 2 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:2. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:2.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 3 or a variant thereof comprising one or more conservative amino acid substitutions. The targeting sequence (e.g. the muscle cell targeting sequence) may be SEQ ID NO: 3 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:3. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:3.

The targeting sequence may be any sequence listed in Table 1 or a variant thereof comprising one or more conservative amino acid substitutions.

**Table 1. Exemplary targeting sequences.**

| SEQ ID NO | Sequence | Peptide name |
|---|---|---|
| 1 | ASSLNIA | MD1 |
| 2 | RRQPPRSISSHP | MD2 |
| 3 | SKTFNTHPQSTP | MD3 |

Thus, the invention provides a nanoparticle (or a non-viral transfection complex) comprising:
(a) a cargo
(b) a lipid component; and
(c) a targeting peptide comprising a muscle cell targeting sequence and cargo-binding component, wherein the muscle cell targeting sequence is SEQ ID NO: 1 or a variant thereof comprising one or more conservative amino acid substitutions.

The invention also provides a nanoparticle (or a non-viral transfection complex) comprising:
(a) a cargo
(b) a lipid component; and
(c) a targeting peptide comprising a muscle cell targeting sequence and cargo-binding component, wherein the muscle cell targeting sequence is SEQ ID NO: 2 or a variant thereof comprising one or more conservative amino acid substitutions.

The invention also provides a nanoparticle (or a non-viral transfection complex) comprising:
(a) a cargo
(b) a lipid component; and
(c) a targeting peptide comprising a muscle cell targeting sequence and cargo-binding component, wherein the muscle cell targeting sequence is SEQ ID NO: 3 or a variant thereof comprising one or more conservative amino acid substitutions.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding component, wherein the muscle cell targeting sequence is SEQ ID NO: 1 or a variant thereof comprising one or more conservative amino acid substitutions.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding component, wherein the muscle cell targeting sequence is SEQ ID NO: 2 or a variant thereof comprising one or more conservative amino acid substitutions.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding component, wherein the muscle cell targeting sequence is SEQ ID NO: 3 or a variant thereof comprising one or more conservative amino acid substitutions.

Preferably, the nanoparticle (e.g. the non-viral transfection complex) comprises:
(a) a cargo, wherein the cargo is a nucleic acid;
(b) a lipid component; and
(c) a targeting peptide comprising a muscle cell targeting sequence and a nucleic acid-binding cationic component, wherein the muscle cell targeting sequence is SEQ ID NO: 1 or a variant thereof comprising one or more conservative amino acid substitutions.

Preferably, the nanoparticle (e.g. the non-viral transfection complex) comprises:
(a) a cargo, wherein the cargo is a nucleic acid;
(b) a lipid component; and
(c) a targeting peptide comprising a muscle cell targeting sequence and a nucleic acid-binding cationic component, wherein the muscle cell targeting sequence is SEQ ID NO: 2 or a variant thereof comprising one or more conservative amino acid substitutions.

Preferably, the nanoparticle (e.g. the non-viral transfection complex) comprises:
(a) a cargo, wherein the cargo is a nucleic acid;
(b) a lipid component; and
(c) a targeting peptide comprising a muscle cell targeting sequence and a nucleic acid-binding cationic component, wherein the muscle cell targeting sequence is SEQ ID NO: 3 or a variant thereof comprising one or more conservative amino acid substitutions.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding polycationic component, wherein the muscle cell targeting sequence is SEQ ID NO: 1 or a variant thereof comprising one or more conservative amino acid substitutions.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding polycationic component, wherein the muscle cell targeting sequence is SEQ ID NO: 2 or a variant thereof comprising one or more conservative amino acid substitutions.

The nanoparticle (e.g. the non-viral transfection complex) may comprise:
(a) a cargo, wherein the cargo is a nucleic acid;
(b) a lipid component; and
(c) a targeting peptide comprising a muscle cell targeting sequence and a nucleic acid-binding polycationic component, wherein the muscle cell targeting sequence is SEQ ID NO: 3 or a variant thereof comprising one or more conservative amino acid substitutions.

The nanoparticle (e.g. the non-viral transfection complex) may comprise:
(a) a cargo, wherein the cargo is a nucleic acid, wherein the nucleic acid is a closed linear DNA molecule;
(b) a lipid component; and
(c) a targeting peptide comprising a muscle cell targeting sequence and a closed linear DNA-binding component (e.g. a closed linear DNA-binding polycationic component), wherein the muscle cell targeting sequence is SEQ ID NO: 1, SEQ ID NO: 2, or SEQ ID NO: 3 or a variant thereof comprising one or more conservative amino acid substitutions.

The nanoparticle (e.g. the non-viral transfection complex) may comprise:
(a) a cargo, wherein the cargo is a nucleic acid, wherein the nucleic acid is a linear DNA molecule comprising one or more nuclease-resistant nucleotides (e.g. phosphorothioated nucleotides);
(b) a lipid component; and
(c) a targeting peptide comprising a muscle cell targeting sequence and a linear DNA-binding component (e.g. a linear DNA-binding polycationic component), wherein the muscle cell targeting sequence is SEQ ID NO: 1, SEQ ID NO: 2, or SEQ ID NO: 3 or a variant thereof comprising one or more conservative amino acid substitutions.

The targeting peptide may comprise at least 4, at least 5, at least 6, at least 7, at least 8, at least 9, or at least 10 amino acids. The targeting sequence (e.g. the muscle cell targeting sequence) may comprise 4-100, 5-70, 6-50, or 7-40 amino acids.

The targeting peptide may comprise at least 4, at least 5, at least 6, at least 7, or at least 8 contiguous amino acids of SEQ ID NO: 4 or a variant thereof comprising one or more conservative amino acid substitutions. The targeting peptide may be SEQ ID NO: 4 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:4. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:4.

The targeting peptide may comprise at least 6, at least 7, or at least 8, at least 9, at least 10 contiguous amino acids of SEQ ID NO: 5 or a variant thereof comprising one or more conservative amino acid substitutions. The targeting peptide may be SEQ ID NO: 5 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:5. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:5.

The targeting peptide may comprise at least 8, at least 9, at least 10, at least 11, at least 12, at least 13, or at least 14 contiguous amino acids of SEQ ID NO: 6 or a variant thereof comprising one or more conservative amino acid substitutions. The targeting peptide may be SEQ ID NO: 6 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:6. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:6.

The targeting peptide may comprise at least 20, at least 21, at least 22, at least 23, least 24, at least 25, at least 26, at least 27, at least 28, at least 29, or at least 30 contiguous amino acids of SEQ ID NO: 7 or a variant thereof comprising one or more conservative amino acid substitutions. The targeting peptide may be SEQ ID NO: 7 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:7. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:7.

The targeting peptide may comprise at least 34, at least 35, at least 36, at least 37, least 38, at least 39, at least 40, at least 41, at least 42, at least 43, or at least 44 contiguous amino acids of SEQ ID NO: 8 or a variant thereof comprising one or more conservative amino acid substitutions. The targeting peptide may be SEQ ID NO: 8 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:8. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:8.

The targeting peptide may comprise at least 18, at least 19, at least 20, at least 21, at least 22, at least 23, at least 24, at least 25, or at least 26 contiguous amino acids of SEQ ID NO: 9 or a variant thereof comprising one or more conservative amino acid substitutions. The targeting peptide may be SEQ ID NO: 9 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:9. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:9.

The targeting peptide may comprise at least 32, at least 33, at least 34, at least 35, at least 36, at least 37, at least 38, at least 39, or at least 40 contiguous amino acids of SEQ ID NO: 10 or a variant thereof comprising one or more conservative amino acid substitutions. The targeting peptide may be SEQ ID NO: 10 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:10. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:10.

The targeting peptide may comprise at least 4, at least 5, at least 6, at least 7, or at least 8 contiguous amino acids of SEQ ID NO: 11 or a variant thereof comprising one or more conservative amino acid substitutions. The targeting peptide may be SEQ ID NO: 11 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:11. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:11.

The targeting peptide may comprise at least 16, at least 17, at least 18, at least 19, at least 20, at least 21, at least 22, at least 23, or at least 24 contiguous amino acids of SEQ ID NO: 12 or a variant thereof comprising one or more conservative amino acid substitutions. The targeting peptide may be SEQ ID NO: 12 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:12. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:12.

The targeting peptide may comprise at least 30, at least 31, at least 32, at least 33, at least 34, at least 35, at least 36, at least 37, or at least 38 contiguous amino acids of SEQ ID NO: 13 or a variant thereof comprising one or more conservative amino acid substitutions. The targeting peptide may be SEQ ID NO: 13 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:13. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:13.

The targeting peptide may comprise at least 7, at least 8, at least 9, at least 10, at least 11, at least 12, or at least 13, contiguous amino acids of SEQ ID NO: 14 or a variant thereof comprising one or more conservative amino acid substitutions. The targeting peptide may be SEQ ID NO: 14 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:14. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:14.

The targeting peptide may comprise at least 9, at least 10, at least 11, at least 12, at least 13, at least 14, or at least 15 contiguous amino acids of SEQ ID NO: 15 or a variant thereof comprising one or more conservative amino acid substitutions. The targeting peptide may be SEQ ID NO: 15 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:15. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:15.

The targeting peptide may comprise at least 11, at least 12, at least 13, at least 14, at least 15, at least 16, at least 17, at least 18, or at least 19 contiguous amino acids of SEQ ID NO: 16 or a variant thereof comprising one or more conservative amino acid substitutions. The targeting peptide may be SEQ ID NO: 16 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:16. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:16.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 17 or a variant thereof comprising one or more conservative amino acid substitutions. The targeting peptide may be SEQ ID NO: 17 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:17. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:17.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 18 or a variant thereof comprising one or more conservative amino acid substitutions. The targeting peptide may be SEQ ID NO: 18 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:18. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:18.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 19 or a variant thereof comprising one or more conservative amino acid substitutions. The targeting peptide may be SEQ ID NO: 19 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:19. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:19.

The targeting peptide may comprise at least 34, least 35, at least 36, at least 37, least 38, at least 39, at least 40, at least 41, at least 42, at least 43, at least 44, or at least 45 contiguous amino acids of SEQ ID NO: 20 or a variant thereof comprising one or more conservative amino acid substitutions.

The targeting peptide may be SEQ ID NO: 20 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:20. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:20.

The targeting peptide may comprise at least 6, at least 7, at least 8, at least 9, or at least 10, at least 11, at least 12, or at least 13 contiguous amino acids of SEQ ID NO: 21 or a variant thereof comprising one or more conservative amino acid substitutions. The targeting peptide may be SEQ ID NO: 21 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 21. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:21.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 22 or a variant thereof comprising one or more conservative amino acid substitutions. The targeting peptide may be SEQ ID NO: 22 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:22. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:22.

The targeting peptide may comprise at least 33, least 34, at least 35, least 36, at least 37, at least 38, least 39, at least 40, at least 41, at least 42, at least 43, or at least 44 contiguous amino acids of SEQ ID NO: 23 or a variant thereof comprising one or more conservative amino acid substitutions. The targeting peptide may be SEQ ID NO: 23 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:23. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:23.

The targeting peptide may comprise at least 6, at least 7, at least 8, at least 9, or at least 10, at least 11, at least 12, or at least 13 contiguous amino acids of SEQ ID NO: 24 or a variant thereof comprising one or more conservative amino acid substitutions. The targeting peptide may be SEQ ID NO: 24 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:24. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:24.

The targeting peptide may comprise at least 8, at least 9, or at least 10, at least 11, at least 12, at least 13, at least 14, or at least 15 contiguous amino acids of SEQ ID NO: 25 or a variant thereof comprising one or more conservative amino acid substitutions. The targeting peptide may be SEQ ID NO: 25 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:25. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:25.

The targeting peptide may comprise at least 11, at least 12, at least 13, at least 14, at least 15, at least 16, at least 17, at least 18, or at least 19 contiguous amino acids of SEQ ID NO: 26 or a variant thereof comprising one or more conservative amino acid substitutions. The targeting peptide may be SEQ ID NO: 26 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:26. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:26.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 27 or a variant thereof comprising one or more conservative amino acid substitutions. The targeting peptide may be SEQ ID NO: 27 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:27. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:27.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 28 or a variant thereof comprising one or more conservative amino acid substitutions. The targeting peptide may be SEQ ID NO: 28 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:28. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:28.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 29 or a variant thereof comprising one or more conservative amino acid substitutions. The targeting peptide may be SEQ ID NO: 29 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:29. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:29.

The targeting peptide may comprise at least 35, at least 36, at least 37, least 38, at least 39, at least 40, at least 41, at least 42, at least 43, at least 44, or at least 45 contiguous amino acids of SEQ ID NO: 30 or a variant thereof comprising one or more conservative amino acid substitutions. The targeting peptide may be SEQ ID NO: 30 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:30. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:30.

The targeting peptide may comprise at least 6, at least 7, at least 8, at least 9, or at least 10, at least 11, at least 12, or at least 13 contiguous amino acids of SEQ ID NO: 31 or a variant thereof comprising one or more conservative amino acid substitutions. The targeting peptide may be SEQ ID NO: 31 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:31. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:31.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 32 or a variant thereof comprising one or more conservative amino acid substitutions. The targeting peptide may be SEQ ID NO: 32 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:32. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:32.

The targeting peptide may comprise at least 32, least 33, at least 34, least 35, at least 36, at least 37, least 38, at least 39, at least 40, at least 41, at least 42, or at least 43 contiguous amino acids of SEQ ID NO: 33 or a variant thereof comprising one or more conservative amino acid substitutions. The targeting peptide may be SEQ ID NO: 33 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:33. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:33.

By "sequence identity" or "sequence similarity" is meant that the identity or similarity, respectively, between two or more amino acid sequences, or two or more nucleotide sequences, is expressed in terms of the identity or similarity between the sequences. Sequence identity can be measured in terms of "percentage (%) identity," in which a higher percentage indicates greater identity shared between the sequences. Sequence similarity can be measured in terms of percentage similarity (which takes into account conservative amino acid substitutions); the higher the percentage, the more similarity shared between the sequences.

The peptides described herein may comprise conservative amino acid substitutions at one or more amino acid residues, e.g. at essential or non-essential amino acid residues. A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagines, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The targeting peptide may comprise any one of the sequences of Table 2.

**Table 2. Exemplary targeting peptides.**

| SEQ ID NO | Sequence | Peptide name |
|---|---|---|
| 4 | CASSLNIAC | |
| 5 | GACASSLNIAC | |
| 6 | RVRRGACASSLNIAC | |
| 7 | KKKKKKKKKKKKKKKKRVRRGACASSLNIAC | MD1CC |
| 8 | KKKKKKKKKKKKKKKKKKKKKKKKKKKKKKRVRRGACASSLNIAC | |
| 9 | KKKKKKKKKKKKKKKKGACASSLNIAC | MD1C |
| 10 | KKKKKKKKKKKKKKKKKKKKKKKKKKKKKKGACASSLNIAC | |
| 11 | GAASSLNIA | |
| 12 | KKKKKKKKKKKKKKKKGAASSLNIA | MD1L |
| 13 | KKKKKKKKKKKKKKKKKKKKKKKKKKKKKKGAASSLNIA | |
| 14 | CRRQPPRSISSHPC | |
| 15 | GACRRQPPRSISSHPC | |
| 16 | RVRRGACRRQPPRSISSHPC | |
| 17 | KKKKKKKKKKKKKKKKRVRRGACRRQPPRSISSHPC | MD2CC |
| 18 | KKKKKKKKKKKKKKKKKKKKKKKKKKKKKKRVRRGACRRQPPRSISSHPC | |
| 19 | KKKKKKKKKKKKKKKKGACRRQPPRSISSHPC | MD2C |
| 20 | KKKKKKKKKKKKKKKKKKKKKKKKKKKKKKGACRRQPPRSISSHPC | |
| 21 | GARRQPPRSISSHP | |
| 22 | KKKKKKKKKKKKKKKKGARRQPPRSISSHP | MD2L |
| 23 | KKKKKKKKKKKKKKKKKKKKKKKKKKKKKKGARRQPPRSISSHP | |
| 24 | CSKTFNTHPQSTPC | |
| 25 | GACSKTFNTHPQSTPC | |
| 26 | RVRRGACSKTFNTHPQSTPC | |
| 27 | KKKKKKKKKKKKKKKKRVRRGACSKTFNTHPQSTPC | MD3CC |
| 28 | KKKKKKKKKKKKKKKKKKKKKKKKKKKKKKRVRRGACSKTFNTHPQSTPC | |
| 29 | KKKKKKKKKKKKKKKKGACSKTFNTHPQSTPC | MD3C |
| 30 | KKKKKKKKKKKKKKKKKKKKKKKKKKKKKKGACSKTFNTHPQSTPC | |
| 31 | GASKTFNTHPQSTP | |
| 32 | KKKKKKKKKKKKKKKKGASKTFNTHPQSTP | MD3L |
| 33 | KKKKKKKKKKKKKKKKKKKKKKKKKKKKKKGASKTFNTHPQSTP | |

The targeting peptide may comprise a cyclic region, a branched region, and/or a linear region.

The targeting peptide comprising the cyclic region may be formed by the provision of at least two cysteine residues in the peptide, thus enabling the formation of a disulphide bond. Thus, the targeting peptide may comprise two or more cysteine residues that are capable of forming one or more disulphide bond(s). Preferably, the two or more cysteine residues flank the targeting sequence. For example, if the targeting sequence is ASSLNIA (SEQ ID NO: 1), the targeting peptide may comprise a sequence: CASSLNIAC (SEQ ID NO: 4).

The targeting peptide may comprise a linker. The linker may be cleavable or non-cleavable.

The linker may comprise at least 1, at least 2, at least 3, at least 4, at least 5, at least 6, at least 7, at least 8, at least 9, at least 10, at least 11 or at least 12 amino acids. The linker may comprise 2-10 amino acids or 4-8 amino acids. The amino acids may be naturally occurring or non-naturally occurring. They may have L- or D- configuration. The amino acids may be the same or different. The use of multiple lysine residues (or other cationic amino acids suitable for use in the cargo-binding polycationic component) should generally be avoided in the linker as oligo-lysine sequences have activity as a cargo-binding polycationic component.

The linker may comprise a cleavable portion that is susceptible to cleavage within a cell. The linker that comprises a cleavable portion that is susceptible to cleavage within a cell may be susceptible to cleavage within the endosome, lysosome, and/or cytoplasm of a cell. The expression "susceptible to cleavage" refers to a linker that is susceptible to cleavage over a timescale during which the remaining elements of the targeting peptide are intact. Thus, the linker may be cleaved more rapidly than the cellular peptide-degradation pathways take effect. The cleavable portion may comprise from 3 to 6 amino acids, for example 4 amino acids. The linker may include the amino acid sequence RVRR (SEQ ID NO: 34) as a cleavable portion. The amino acid sequence RVRR is susceptible to enzymatic cleavage by the endosomal protease furin. The cleavable portion of the linker may be attached to a cargo-binding component. The cleavable portion of the linker may be cleavable by a protease. The protease may be cathepsin (e.g. serine, cysteine, aspartic-type), furin, a lysosomal protease or an endosomal protease.

The targeting peptide may comprise a spacer. The spacer may be either a peptide, that is to say, it comprises amino acid residues, or a polyethyleneglycol group, or a mixture of the two. The amino acids may be naturally occurring or non-naturally occurring. They may have L- or D-configuration. The spacer may have one or more amino acids. The spacer may comprise at least 1, at least 2, at least 3, at least 4, at least 5, at least 6, at least 7, at least 8, at least 9, at least 10, at least 11 or at least 12 amino acids.

The spacer may comprise 1-7 amino acids, preferably 2-5 amino acids. The amino acids may be the same or different. The spacer may comprise the dipeptide glycine-glycine (GG), glycine-alanine (GA) or alanine-alanine (AA). The spacer may comprise a hydrophobic spacer. The spacer may include the amino acid sequence XSX in which X is c-aminocaproic acid (c-Ahx) also known as 6-aminohexanoic acid, a synthetic, i.e. non-naturally occurring, amino acid. Aminocaproic acid functions as a hydrophobic spacer. The spacer may comprise GG, GA, AA, XSXGG (SEQ ID NO: 35), XSXGA (SEQ ID NO: 36) or XSXAA (SEQ ID NO: 38). Preferably, the spacer comprises GA or GG. The spacer may be located at the end of the linker in a targeting peptide. The spacer may be attached to a targeting sequence or a targeting sequence flanked by cysteine residues. Preferably, the spacer links the linker and the targeting sequence (which is optionally flanked by the cysteine residues).

The targeting peptide may have a structure: A-B-C-D, wherein component A is a cargo-binding component, component B is a linker, component C is a spacer and component D is a targeting sequence (optionally flanked by the cysteine residues).

The targeting peptide may have a structure: A-B-D, wherein component A is a cargo-binding component, component B is a linker and component D is a targeting sequence (optionally flanked by the cysteine residues).

The targeting peptide may have a structure: A-D, wherein component A is a cargo-binding component and component D is a targeting sequence (optionally flanked by the cysteine residues).

The nanoparticle (e.g. the non-viral transfection complex) may have a particle size of less than 500 nm, for example less than 250 nm or less than 100 nm. In a population of particles there will be some variation in particle size but the above criteria will be taken as met if at least 70%, at least 80% or at least 90% of the particles are of less than 500 nm, for example less than 250nm or less than 100 nm. Preferably, in a population of particles, at least 80% of the particles are less than 500 nm, for example less than 250nm or less than 100 nm.

The nanoparticle (e.g. the non-viral transfection complex) may have a charge ratio (i.e. Nitrogen/Phosphate (N/P) molar ratio) of 2.0-13.0, 3.0-12.0, 4.0-11.0, 7.0-13.0, 7.2-13.0, 7.5-13.0, or 8.0-13.0. The nanoparticle (e.g. the non-viral transfection complex) may have a charge ratio (i.e. N/P ratio) of about 4.2, about 4.5, about 4.7, about 5.2, about 5.5, about 5.7 about 6.5, about 7.5, about 8.5, about 9.5 or about 10.5. Preferably, the nanoparticle has a charge ratio (i.e. N/P ratio) of 7.0-11.0 or 3.0-8.0.

If the cargo in the nanoparticle (e.g. the non-viral transfection complex) is a plasmid DNA, the nanoparticle may have a charge ratio (i.e. Nitrogen/Phosphate (N/P) molar ratio) of 4.0-9.0, 5.0-8.0, or 6.0-8.0. Preferably, the nanoparticle has a charge ratio of 6.0-8.0.

If the cargo in the nanoparticle (e.g. the non-viral transfection complex) is a linear DNA molecule (e.g. a closed linear DNA molecule, such as a covalently-closed linear DNA molecule), the nanoparticle may have a charge ratio (i.e. Nitrogen/Phosphate (N/P) molar ratio) of 2.0-13.0, 3.0-11.0, 4.0-9.0, or 4.0-7.0. Preferably, the nanoparticle has a charge ratio of 4.0-7.0.

If the cargo-binding component is a nucleic acid-binding polycationic component comprising 16 lysine residues, the nanoparticle may have a charge ratio (i.e. N/P ratio) of 2.0-13.0, 3.0-11.0, 4.0-9.0, or 4.0-7.0. Preferably, the nanoparticle has a charge ratio of 4.0-7.0. For example, if the cargo-binding component is a nucleic acid-binding polycationic component comprising 16 lysine residues, the nanoparticle may have a charge ratio (i.e. N/P ratio) of about 4.5, about 5.0, about 5.5, about 6.0 or about 6.5.

If the cargo-binding component is a nucleic acid-binding polycationic component comprising 30 lysine residues, the nanoparticle may have a charge ratio (i.e. N/P ratio) of 3.0-12.0, 3.0-10.0, 3.0-8.0, or 4.0-8.0. For example, if the cargo-binding component is a nucleic acid-binding polycationic component comprising 30 lysine residues, the nanoparticle may have a charge ratio (i.e. N/P ratio) of about 4.2, about 4.5, about 4.7, about 5.2, about 5.5, about 5.7 about 6.5, or about 7.5 .

If the cargo in the nanoparticle (e.g. the non-viral transfection complex) is a protein, the nanoparticle may have a charge ratio (i.e. Nitrogen/Phosphate (N/P) molar ratio) of 2.0-13.0, 3.0-11.0, 4.0-9.0, or 4.0-7.0. Preferably, the nanoparticle has a charge ratio of 4.0-7.0.

If the cargo in the nanoparticle (e.g. the non-viral transfection complex) is an mRNA, the nanoparticle may have a charge ratio (i.e. Nitrogen/Phosphate (N/P) molar ratio) of 2.0-13.0, 3.0-11.0, 4.0-9.0, or 4.0-7.0. Preferably, the nanoparticle has a charge ratio of 4.0-7.0.

The charge ratio (N/P ratio) is calculated from the molar amount of each free amine group(s) (N) in the components of the nanoparticle to the phosphate groups (P) in the components of the nanoparticle. For example, the free amine group(s) may come from the targeting peptide and the lipid component and the phosphate group(s) may come from the phosphate groups in the cargo (e.g. DNA molecule) (P). The charge ratio is typically driven by the mass of the targeting peptide.

The term "about" as used herein for numerical parameters refers to a value within 10% of the underlying parameter (i.e. plus or minus 10%). For example, a charge ratio of "about 4.5" can include charge ratios between 4.1 - 5.0, including charge ratios 4.1 and 5.0.

The nanoparticle (e.g. the non-viral delivery complex) may deliver the cargo to a muscle cell with a transfection efficiency of at least 5%, at least 10%, at least 15%, at least 20%, at least 25%, at least 30%, at least 35%, at least 40%, at least 50%, at least 52%, at least 55%, at least 60%, at least 65%, at least 70%, at least 75%, at least 80%, at least 85%, at least 90%, at least 95%, or at least 98%. Preferably, the transfection efficiency is at least 15%.

### 2. Targeting peptides

The invention provides a targeting peptide comprising a targeting sequence.

Preferably, the targeting sequence is a muscle cell targeting sequence. The targeting peptide may be suitable for use in the nanoparticle (e.g. the non-viral transfection complex) described herein. The targeting peptide may comprise a cargo-binding component.

The invention provides a targeting peptide comprising:
(a) a muscle cell targeting sequence; and
(b) a cargo-binding component.

The muscle cell targeting sequence may be a skeletal muscle cell targeting sequence, a cardiac muscle cell targeting sequence, or a smooth muscle cell targeting sequence. Preferably, the muscle cell targeting sequence is a skeletal muscle cell targeting sequence.

The muscle cell targeting sequence may preferentially bind to a skeletal muscle cell over the smooth muscle cell or the cardiac muscle cell. That is to say that the muscle cell targeting sequence is a skeletal muscle cell-specific targeting sequence. The muscle cell targeting sequence may preferentially bind to the cardiac muscle cell over the smooth muscle cell. That is to say that the muscle cell targeting sequence is a cardiac muscle cell-specific targeting sequence.

The targeting sequence (e.g. the muscle cell targeting sequence) may comprise at least 3, at least 4, at least 5, at least 6, at least 7, at least 8, at least 9, or at least 10 amino acids. The targeting sequence (e.g. the muscle cell targeting sequence) may comprise 3-30, 4-20, 5-17, 6-15 or 7-14 amino acids. Preferably, the targeting sequence comprises 7-14 amino acids. For example, the targeting sequence may comprise 7 or 12 amino acids.

The targeting sequence (e.g. the muscle cell targeting sequence) may comprise at least 3, at least 4, at least 5 or at least 6 contiguous amino acids of SEQ ID NO: 1 or a variant thereof comprising one or more conservative amino acid substitutions. The targeting sequence (e.g. the muscle cell targeting sequence) may be SEQ ID NO: 1 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:1. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:1.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 2 or a variant thereof comprising one or more conservative amino acid substitutions. The targeting sequence (e.g. the muscle cell targeting sequence) may be SEQ ID NO: 2 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:2. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:2.

The targeting sequence (e.g. the muscle cell targeting sequence) may comprise at least 4, at least 5, at least 6, at least 7, at least 8, at least 9, at least 10 or at least 11 contiguous amino acids of SEQ ID NO: 3 or a variant thereof comprising one or more conservative amino acid substitutions. The targeting sequence (e.g. the muscle cell targeting sequence) may be SEQ ID NO: 3 or a variant thereof comprising one or more conservative amino acid substitutions. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:3. The targeting sequence (e.g. the muscle cell targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:3.

Thus, the invention provides a targeting peptide comprising a muscle cell targeting sequence and cargo-binding component, wherein the muscle cell targeting sequence is SEQ ID NO: 1 or a variant thereof comprising one or more conservative amino acid substitutions.

The invention also provides a targeting peptide comprising a muscle cell targeting sequence and cargo-binding component, wherein the muscle cell targeting sequence is SEQ ID NO: 2 or a variant thereof comprising one or more conservative amino acid substitutions.

The invention provides a targeting peptide comprising a muscle cell targeting sequence and cargo-binding component, wherein the muscle cell targeting sequence is SEQ ID NO: 3 or a variant thereof comprising one or more conservative amino acid substitutions.

The targeting peptide may comprise a muscle cell targeting sequence and a nucleic acid-binding component, wherein the muscle cell targeting sequence is SEQ ID NO: 1 or a variant thereof comprising one or more conservative amino acid substitutions.

The targeting peptide may comprise a muscle cell targeting sequence and a nucleic acid-binding component, wherein the muscle cell targeting sequence is SEQ ID NO: 2 or a variant thereof comprising one or more conservative amino acid substitutions.

The targeting peptide may comprise a muscle cell targeting sequence and a nucleic acid-binding component, wherein the muscle cell targeting sequence is SEQ ID NO: 3 or a variant thereof comprising one or more conservative amino acid substitutions.

The targeting peptide may comprise a muscle cell targeting sequence and a nucleic acid-binding cationic component, wherein the muscle cell targeting sequence is SEQ ID NO: 1 or a variant thereof comprising one or more conservative amino acid substitutions.

The targeting peptide may comprise a muscle cell targeting sequence and a nucleic acid-binding cationic component, wherein the muscle cell targeting sequence is SEQ ID NO: 2 or a variant thereof comprising one or more conservative amino acid substitutions.

The targeting peptide may comprise a muscle cell targeting sequence and a nucleic acid-binding cationic component, wherein the muscle cell targeting sequence is SEQ ID NO: 3 or a variant thereof comprising one or more conservative amino acid substitutions.

The targeting peptide may comprise a muscle cell targeting sequence and a nucleic acid-binding polycationic component, wherein the muscle cell targeting sequence is SEQ ID NO: 1 or a variant thereof comprising one or more conservative amino acid substitutions.

The targeting peptide may comprise a muscle cell targeting sequence and a nucleic acid-binding polycationic component, wherein the muscle cell targeting sequence is SEQ ID NO: 2 or a variant thereof comprising one or more conservative amino acid substitutions.

The targeting peptide may comprise a muscle cell targeting sequence and a nucleic acid-binding polycationic component, wherein the muscle cell targeting sequence is SEQ ID NO: 3 or a variant thereof comprising one or more conservative amino acid substitutions.

The targeting peptide may comprise a muscle cell targeting sequence and a closed linear DNA-binding component (e.g. a closed linear DNA-binding polycationic component), wherein the muscle cell targeting sequence is SEQ ID NO: 1, SEQ ID NO: 2, or SEQ ID NO: 3 or a variant thereof comprising one or more conservative amino acid substitutions.

The targeting peptide may comprise a muscle cell targeting sequence and a linear DNA-binding component (e.g. a linear DNA-binding polycationic component), wherein the muscle cell targeting sequence is SEQ ID NO: 1, SEQ ID NO: 2, or SEQ ID NO: 3 or a variant thereof comprising one or more conservative amino acid substitutions.

The targeting sequence may comprise at least 3, at least 4, at least 5, at least 6, at least 7, at least 8, at least 9, at least 10, at least 11, at least 12, at least 13 or at least 14 amino acids. The targeting sequence may comprise 3-30, 4-20, 5-17, 6-15 or 7-14 amino acids. Preferably, the targeting sequence comprises 7-14 amino acids. For example, the targeting sequence may comprise 7 or 12 amino acids.

The targeting peptide may comprise at least 4, at least 5, at least 6, at least 7, or at least 8 contiguous amino acids of SEQ ID NO: 4 or a variant thereof comprising one or more conservative amino acid substitutions. The targeting peptide may be SEQ ID NO: 4 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:4. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:4.

The targeting peptide may comprise at least 6, at least 7, or at least 8, at least 9, at least 10 contiguous amino acids of SEQ ID NO: 5 or a variant thereof comprising one or more conservative amino acid substitutions. The targeting peptide may be SEQ ID NO: 5 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:5. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:5.

The targeting peptide may comprise at least 8, at least 9, at least 10, at least 11, at least 12, at least 13, or at least 14 contiguous amino acids of SEQ ID NO: 6 or a variant thereof comprising one or more conservative amino acid substitutions. The targeting peptide may be SEQ ID NO: 6 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:6. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:6.

The targeting peptide may comprise at least 20, at least 21, at least 22, at least 23, least 24, at least 25, at least 26, at least 27, at least 28, at least 29, or at least 30 contiguous amino acids of SEQ ID NO: 7 or a variant thereof comprising one or more conservative amino acid substitutions. The targeting peptide may be SEQ ID NO: 7 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:7. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:7.

The targeting peptide may comprise at least 34, at least 35, at least 36, at least 37, least 38, at least 39, at least 40, at least 41, at least 42, at least 43, or at least 44 contiguous amino acids of SEQ ID NO: 8 or a variant thereof comprising one or more conservative amino acid substitutions. The targeting peptide may be SEQ ID NO: 8 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:8. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:8.

The targeting peptide may comprise at least 18, at least 19, at least 20, at least 21, at least 22, at least 23, at least 24, at least 25, or at least 26 contiguous amino acids of SEQ ID NO: 9 or a variant thereof comprising one or more conservative amino acid substitutions. The targeting peptide may be SEQ ID NO: 9 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:9. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:9.

The targeting peptide may comprise at least 32, at least 33, at least 34, at least 35, at least 36, at least 37, at least 38, at least 39, or at least 40 contiguous amino acids of SEQ ID NO: 10 or a variant thereof comprising one or more conservative amino acid substitutions. The targeting peptide may be SEQ ID NO: 10 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:10. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:10.

The targeting peptide may comprise at least 4, at least 5, at least 6, at least 7, or at least 8 contiguous amino acids of SEQ ID NO: 11 or a variant thereof comprising one or more conservative amino acid substitutions. The targeting peptide may be SEQ ID NO: 11 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:11. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:11.

The targeting peptide may comprise at least 16, at least 17, at least 18, at least 19, at least 20, at least 21, at least 22, at least 23, or at least 24 contiguous amino acids of SEQ ID NO: 12 or a variant thereof comprising one or more conservative amino acid substitutions. The targeting peptide may be SEQ ID NO: 12 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:12. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:12.

The targeting peptide may comprise at least 30, at least 31, at least 32, at least 33, at least 34, at least 35, at least 36, at least 37, or at least 38 contiguous amino acids of SEQ ID NO: 13 or a variant thereof comprising one or more conservative amino acid substitutions. The targeting peptide may be SEQ ID NO: 13 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:13. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:13.

The targeting peptide may comprise at least 7, at least 8, at least 9, at least 10, at least 11, at least 12, or at least 13, contiguous amino acids of SEQ ID NO: 14 or a variant thereof comprising one or more conservative amino acid substitutions. The targeting peptide may be SEQ ID NO: 14 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:14. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:14.

The targeting peptide may comprise at least 9, at least 10, at least 11, at least 12, at least 13, at least 14, or at least 15 contiguous amino acids of SEQ ID NO: 15 or a variant thereof comprising one or more conservative amino acid substitutions. The targeting peptide may be SEQ ID NO: 15 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:15. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:15.

The targeting peptide may comprise at least 11, at least 12, at least 13, at least 14, at least 15, at least 16, at least 17, at least 18, or at least 19 contiguous amino acids of SEQ ID NO: 16 or a variant thereof comprising one or more conservative amino acid substitutions. The targeting peptide may be SEQ ID NO: 16 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:16. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:16.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 17 or a variant thereof comprising one or more conservative amino acid substitutions. The targeting peptide may be SEQ ID NO: 17 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:17. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:17.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 18 or a variant thereof comprising one or more conservative amino acid substitutions. The targeting peptide may be SEQ ID NO: 18 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:18. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:18.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 19 or a variant thereof comprising one or more conservative amino acid substitutions. The targeting peptide may be SEQ ID NO: 19 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:19. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:19.

The targeting peptide may comprise at least 34, least 35, at least 36, at least 37, least 38, at least 39, at least 40, at least 41, at least 42, at least 43, at least 44, or at least 45 contiguous amino acids of SEQ ID NO: 20 or a variant thereof comprising one or more conservative amino acid substitutions. The targeting peptide may be SEQ ID NO: 20 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:20. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:20.

The targeting peptide may comprise at least 6, at least 7, at least 8, at least 9, or at least 10, at least 11, at least 12, or at least 13 contiguous amino acids of SEQ ID NO: 21 or a variant thereof comprising one or more conservative amino acid substitutions. The targeting peptide may be SEQ ID NO: 21 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 21. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:21.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 22 or a variant thereof comprising one or more conservative amino acid substitutions. The targeting peptide may be SEQ ID NO: 22 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:22. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:22.

The targeting peptide may comprise at least 33, least 34, at least 35, least 36, at least 37, at least 38, least 39, at least 40, at least 41, at least 42, at least 43, or at least 44 contiguous amino acids of SEQ ID NO: 23 or a variant thereof comprising one or more conservative amino acid substitutions. The targeting peptide may be SEQ ID NO: 23 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:23. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:23.

The targeting peptide may comprise at least 6, at least 7, at least 8, at least 9, or at least 10, at least 11, at least 12, or at least 13 contiguous amino acids of SEQ ID NO: 24 or a variant thereof comprising one or more conservative amino acid substitutions. The targeting peptide may be SEQ ID NO: 24 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:24. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:24.

The targeting peptide may comprise at least 8, at least 9, or at least 10, at least 11, at least 12, at least 13, at least 14, or at least 15 contiguous amino acids of SEQ ID NO: 25 or a variant thereof comprising one or more conservative amino acid substitutions. The targeting peptide may be SEQ ID NO: 25 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:25. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:25.

The targeting peptide may comprise at least 11, at least 12, at least 13, at least 14, at least 15, at least 16, at least 17, at least 18, or at least 19 contiguous amino acids of SEQ ID NO: 26 or a variant thereof comprising one or more conservative amino acid substitutions. The targeting peptide may be SEQ ID NO: 26 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:26. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:26.

The targeting peptide may comprise at least 21, at least 22, at least 23, least 24, at least 25, at least 26, at least 27, at least 28, at least 29, at least 30, at least 31, at least 32, at least 33, at least 34,or at least 35 contiguous amino acids of SEQ ID NO: 27 or a variant thereof comprising one or more conservative amino acid substitutions. The targeting peptide may be SEQ ID NO: 27 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:27. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:27.

The targeting peptide may comprise at least 35, at least 36, at least 37, least 38, at least 39, at least 40, at least 41, at least 42, at least 43, at least 44, at least 45, at least 46, at least 47, at least 48,or at least 49 contiguous amino acids of SEQ ID NO: 28 or a variant thereof comprising one or more conservative amino acid substitutions. The targeting peptide may be SEQ ID NO: 28 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:28. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:28.

The targeting peptide may comprise at least 20, least 21, at least 22, at least 23, least 24, at least 25, at least 26, at least 27, at least 28, at least 29, at least 30, or at least 31 contiguous amino acids of SEQ ID NO: 29 or a variant thereof comprising one or more conservative amino acid substitutions. The targeting peptide may be SEQ ID NO: 29 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:29. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:29.

The targeting peptide may comprise at least 35, at least 36, at least 37, least 38, at least 39, at least 40, at least 41, at least 42, at least 43, at least 44, or at least 45 contiguous amino acids of SEQ ID NO: 30 or a variant thereof comprising one or more conservative amino acid substitutions. The targeting peptide may be SEQ ID NO: 30 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:30. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:30.

The targeting peptide may comprise at least 6, at least 7, at least 8, at least 9, or at least 10, at least 11, at least 12, or at least 13 contiguous amino acids of SEQ ID NO: 31 or a variant thereof comprising one or more conservative amino acid substitutions. The targeting peptide may be SEQ ID NO: 31 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:31. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:31.

The targeting peptide may comprise at least 18, least 19, at least 20, least 21, at least 22, at least 23, least 24, at least 25, at least 26, at least 27, at least 28, or at least 29 contiguous amino acids of SEQ ID NO: 32 or a variant thereof comprising one or more conservative amino acid substitutions. The targeting peptide may be SEQ ID NO: 32 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:32. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:32.

The targeting peptide may comprise at least 32, least 33, at least 34, least 35, at least 36, at least 37, least 38, at least 39, at least 40, at least 41, at least 42, or at least 43 contiguous amino acids of SEQ ID NO: 33 or a variant thereof comprising one or more conservative amino acid substitutions. The targeting peptide may be SEQ ID NO: 33 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO:33. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO:33.

By "sequence identity" or "sequence similarity" is meant that the identity or similarity, respectively, between two or more amino acid sequences, or two or more nucleotide sequences, is expressed in terms of the identity or similarity between the sequences. Sequence identity can be measured in terms of "percentage (%) identity," in which a higher percentage indicates greater identity shared between the sequences. Sequence similarity can be measured in terms of percentage similarity (which takes into account conservative amino acid substitutions); the higher the percentage, the more similarity shared between the sequences.

The targeting peptides described herein may comprise conservative amino acid substitutions at one or more amino acid residues, e.g. at essential or non-essential amino acid residues. A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagines, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The targeting peptide may comprise a cyclic region, a branched region, and/or a linear region.

The targeting peptide comprising the cyclic region may be formed by the provision of at least two cysteine residues in the peptide, thus enabling the formation of a disulphide bond. Thus, the targeting peptide may comprise two or more cysteine residues that are capable of forming one or more disulphide bond(s). Preferably, the two or more cysteine residues flank the targeting sequence. For example, if the targeting sequence is SEQ ID NO: 1, the targeting peptide may comprise a sequence of SEQ ID NO: 4.

The targeting peptide may comprise a linker. The linker may be cleavable or non-cleavable.

The invention provides a targeting peptide comprising:
(a) a muscle cell targeting sequence; and
(b) a linker.

The invention provides a targeting peptide comprising:
(a) a muscle cell targeting sequence;
(b) a linker; and
(c) a cargo-binding component.

The linker may comprise at least 1, at least 2, at least 3, at least 4, at least 5, at least 6, at least 7, at least 8, at least 9, at least 10, at least 11 or at least 12 amino acids. The linker may comprise 2-10 amino acids or 4-8 amino acids. The amino acids may be naturally occurring or non-naturally occurring. They may have L- or D- configuration. The amino acids may be the same or different. The use of multiple lysine residues (or other cationic amino acids suitable for use in the cargo-binding polycationic component) should generally be avoided in the linker as oligo-lysine sequences have activity as a cargo-binding polycationic component.

The linker may comprise a cleavable portion that is susceptible to cleavage within a cell. The linker that comprises a cleavable portion that is susceptible to cleavage within a cell may be susceptible to cleavage within the endosome, lysosome, and/or cytoplasm of a cell. The expression "susceptible to cleavage" refers to a linker that is susceptible to cleavage over a timescale during which the remaining elements on the targeting peptide are intact. Thus, the linker may be cleaved more rapidly than the cellular peptide-degradation pathways take effect. The cleavable portion may comprise from 3 to 6 amino acids, for example 4 amino acids. The linker may include the amino acid sequence RVRR (SEQ ID NO: 34) as a cleavable portion. The amino acid sequence RVRR is susceptible to enzymatic cleavage by the endosomal protease furin. The cleavable portion of the linker may be attached to a cargo-binding component. The cleavable portion of the linker may be cleavable by a protease. The protease may be cathepsin (e.g. serine, cysteine, aspartic-type), furin, a lysosomal protease or an endosomal protease.

The targeting peptide may comprise a spacer. The spacer may be either a peptide, that is to say, it comprises amino acid residues, or a polyethyleneglycol group, or a mixture of the two. The amino acids may be naturally occurring or non-naturally occurring. They may have L- or D-configuration. The spacer may have one or more amino acids. The spacer may comprise at least 1, at least 2, at least 3, at least 4, at least 5, at least 6, at least 7, at least 8, at least 9, at least 10, at least 11 or at least 12 amino acids.

The spacer may comprise 1-7 amino acids, preferably 2-5 amino acids. The amino acids may be the same or different. The spacer may comprise the dipeptide glycine-glycine (GG), glycine-alanine (GA) or alanine-alanine (AA). The spacer may comprise a hydrophobic spacer. The spacer may include the amino acid sequence XSX in which X is c-aminocaproic acid (c-Ahx) also known as 6-aminohexanoic acid, a synthetic, i.e. non-naturally occurring, amino acid. Aminocaproic acid functions as a hydrophobic spacer. The spacer may comprise GG, GA, AA, XSXGG (SEQ ID NO: 35), XSXGA (SEQ ID NO: 36) or XSXAA (SEQ ID NO: 38). Preferably, the spacer comprises GA or GG. The spacer may be located at the end of the linker in a targeting peptide. The spacer may be attached to a targeting sequence or a targeting sequence flanked by cysteine residues. Preferably, the spacer links the linker and the targeting sequence (which is optionally flanked by the cysteine residues).

Thus, the invention provides a targeting peptide comprising:
(a) a muscle cell targeting sequence;
(b) a linker; and
(c) a spacer.

Thus, the invention provides a targeting peptide comprising:
(a) a muscle cell targeting sequence;
(b) a linker; and
(c) a cargo-binding component.

The invention provides a targeting peptide comprising:
(a) a muscle cell targeting sequence;
(b) a linker
(c) a spacer; and
(d) a cargo-binding component.

The targeting peptide may have a structure: A-B-C-D, wherein component A is a cargo-binding component, component B is a linker, component C is a spacer and component D is a targeting sequence (optionally flanked by the cysteine residues).

The targeting peptide may have a structure: A-B-D, wherein component A is a cargo-binding component, component B is a linker and component D is a targeting sequence (optionally flanked by the cysteine residues).

The targeting peptide may have a structure: A-D, wherein component A is a cargo-binding component and component D is a targeting sequence (optionally flanked by the cysteine residues).

The cargo-binding component may be a cargo-binding cationic component, a cargo-binding neutral component or a cargo-binding anionic component. The cationic component, the anionic component, and/or the neutral component may be used to establish a desired charge (i.e. a negative/positive ratio) of the nanoparticle. A specific charge (i.e. a negative/positive ratio) may be required to facilitate or enhance cell transfection.

The cargo-binding component may be a biomolecule-binding component. The biomolecule-binding component may be a biomolecule-binding cationic component, a biomolecule-binding neutral component or a biomolecule-binding anionic component. The cargo-binding component may be a small molecule-binding component. The small molecule-binding component may be a small molecule-binding cationic component, a small molecule-binding neutral component or a small molecule-binding anionic component.

The cargo-binding component may be a nucleic acid-binding component. The nucleic acid-binding component may be a nucleic acid-binding cationic component, or a nucleic acid-binding neutral component.

The nucleic acid-binding component may be a DNA-binding component. The DNA-binding component may be a DNA-binding cationic component or a DNA-binding neutral component. The nucleic acid-binding component may be a closed linear DNA-binding component. The closed linear DNA-binding component may be a closed linear DNA-binding cationic component or a closed linear DNA-binding neutral component. The nucleic acid-binding component may be an RNA-binding component. The RNA-binding component may be an RNA-binding cationic component, or an RNA-binding neutral component. The nucleic acid-binding component may be an antisense RNA-binding component. The antisense RNA-binding component may be an antisense RNA-binding cationic component, or an antisense RNA-binding neutral component. The nucleic acid-binding component may be a siRNA-binding component. The siRNA-binding component may be a siRNA-binding cationic component, or a siRNA-binding neutral component. The nucleic acid-binding component may be an mRNA-binding component. The mRNA-binding component may be an mRNA-binding cationic component, or an mRNA-binding neutral component. The nucleic acid-binding component may be a transfer RNA-binding component. The transfer RNA-binding component may be a transfer RNA-binding cationic component, or a transfer RNA-binding neutral component. The nucleic acid-binding component may be a ribosomal RNA-binding component. The ribosomal RNA-binding component may be a ribosomal RNA-binding cationic component, or a ribosomal RNA-binding neutral component. The nucleic acid-binding component may be an snRNA-binding component. The snRNA-binding component may be an snRNA-binding cationic component, or an snRNA-binding neutral component. The nucleic acid-binding component may be a double-stranded RNA-binding component. The double-stranded RNA-binding component may be a double-stranded RNA-binding cationic component, or a double-stranded RNA-binding neutral component. The nucleic acid-binding component may be an miRNA-binding component. The miRNA-binding component may be an miRNA-binding cationic component, or an miRNA-binding neutral component. The nucleic acid-binding component may be a shRNA-binding component. The shRNA-binding component may be a shRNA-binding cationic component, or a shRNA-binding neutral component. The nucleic acid-binding component may be a gRNA-binding component. The gRNA-binding component may be a gRNA-binding cationic component, or a gRNA-binding neutral component. The nucleic acid-binding component may be a samRNA-binding component. The samRNA-binding component may be a samRNA-binding cationic component, or a samRNA-binding neutral component. The nucleic acid-binding component may be a circular RNA-binding component. The circular RNA-binding component may be a circular RNA-binding cationic component, or a circular RNA-binding neutral component.

The cargo-binding component may be a protein-binding component. The protein-binding component may be a protein-binding cationic component, a protein-binding neutral component or a protein-binding anionic component. The protein-binding component may be a Cas9-binding component. The Cas9 binding component may be a Cas9-binding cationic component, a Cas9-binding neutral component or a Cas9-binding anionic component.

The cargo-binding component may be a peptide-binding component. The peptide-binding component may be a peptide-binding cationic component, a peptide-binding neutral component or a peptide-binding anionic component.

The cargo-binding component may be a polypeptide-binding component. The polypeptide-binding component may be a polypeptide-binding cationic component, a polypeptide-binding neutral component or a polypeptide-binding anionic component.

If the cargo is a nucleic acid, the targeting peptide may comprise a nucleic acid-binding cationic component or a nucleic acid- binding neutral component. Thus, the targeting peptide may comprise:
(a) a muscle cell targeting sequence; and
(b) a nucleic acid-binding component.

Preferably, the nucleic acid-binding component is a nucleic acid-binding cationic component (e.g. DNA-binding cationic component).

The cargo-binding cationic component may be a cargo-binding polycationic component, The cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the cargo-binding polycationic component comprises at least 16 or at least 30 cationic monomers.

The cargo-binding cationic component may comprise a lysine, a histidine, or an arginine. The cargo-binding polycationic component may comprise a lysine, a histidine, or an arginine. The cargo-binding polycationic component may comprise an oligolysine (linear or branched), an oligohistidine (linear or branched) or an oligoarginine (linear or branched). For example, the cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the cargo-binding polycationic component comprises at least 16, at least 17 or at least 30 lysine residues. More preferably still, the cargo-binding polycationic component comprises at least 17 lysine residues. The cargo-binding polycationic component may be linear or branched. For example, the cargo-binding linear polycationic component may comprise at least 17 lysine residues. The cargo-binding branched polycationic component may comprise at least 17 lysine residues.

The cargo-binding anionic component maybe be a cargo-binding polyanionic component, The cargo-binding polyanionic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 anionic monomers. The cargo-binding polyanionic component may be linear or branched. For example, the cargo-binding linear polyanionic component may comprise at least 17 monomers. The cargo-binding branched polyanionic component may comprise at least 17 monomers.

The cargo-binding neutral component maybe be a cargo-binding polyneutral component, The cargo-binding polyneutral component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 neutral monomers. The cargo-binding polyneutral component may be linear or branched. For example, the cargo-binding linear polyneutral component may comprise at least 17 monomers. The cargo-binding branched polyneutral component may comprise at least 17 monomers.

The nucleic acid-binding cationic component (e.g. the DNA-binding cationic component) may be a nucleic acid-binding polycationic component (e.g. a DNA-binding polycationic component). The nucleic acid-binding polycationic component may comprise a lysine. The nucleic acid-binding polycationic component may comprise an oligolysine. The nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the nucleic acid-binding polycationic component comprises at least 16, at least 17, or at least 30 lysine residues. More preferably still, the cargo-binding polycationic component comprises at least 17 lysine residues. The nucleic acid-binding polycationic component may be linear or branched. For example, the nucleic acid-binding linear polycationic component may comprise at least 17 lysine residues. The nucleic acid-binding branched polycationic component may comprise at least 17 lysine residues.

The cargo-binding component may be linear or branched. The cargo-binding polycationic component may be linear or branched. The cargo-binding polyanionic component may be linear or branched. The cargo-binding polyneutral component may be linear or branched. For example, the cargo-binding polycationic component may comprise at least 16, at least 17, or at least 30 lysine residues in a linear chain. Alternatively, the cargo-binding polycationic component may comprise at least 16, at least 17, or at least 30 lysine residues in a branched chain. The cargo may be a nucleic acid-binding polycationic component. The nucleic acid-binding polycationic component may be linear or branched. For example, the nucleic acid-binding polycationic component (e.g. the DNA-binding polycationic component) may comprise at least 16, at least 17, or at least 30 lysine residues in a linear chain. Alternatively, the nucleic acid-binding polycationic component (e.g. the DNA-binding polycationic component) may comprise at least 16, at least 17, or at least 30 lysine residues in a branched chain. Thus, the targeting peptide may comprise a muscle cell targeting sequence and a nucleic acid-binding component. The targeting peptide may comprise a muscle cell targeting sequence and a nucleic acid-binding cationic component. The targeting peptide may comprise a muscle cell targeting sequence and a nucleic acid-binding polycationic component (e.g. DNA-binding polycationic component). The targeting peptide may comprise a muscle cell targeting sequence and a closed linear DNA-binding component (e.g. a closed linear DNA-binding polycationic component). The targeting peptide may comprise a muscle cell targeting sequence and a linear DNA-binding component (e.g. a linear DNA-binding polycationic component).

The targeting peptide may comprise:
(a) a muscle cell targeting sequence;
(b) a spacer; and
(c) a nucleic acid-binding component.

The targeting peptide may comprise a muscle cell targeting sequence, a spacer, and a nucleic acid-binding cationic component. The targeting peptide may comprise a muscle cell targeting sequence, a spacer, and a nucleic acid-binding polycationic component. The targeting peptide may comprise a muscle cell targeting sequence, a spacer, and a closed linear DNA-binding component (e.g. a closed linear DNA-binding polycationic component). The targeting peptide may comprise a muscle cell targeting sequence, a spacer, and a linear DNA-binding component (e.g. a linear DNA-binding polycationic component).

The targeting peptide may comprise:
(a) a muscle cell targeting sequence;
(b) a linker; and
(c) a nucleic acid-binding component.

The targeting peptide may comprise a muscle cell targeting sequence, a linker, and a nucleic acid-binding cationic component. The targeting peptide may comprise a muscle cell targeting sequence, a linker, and a nucleic acid-binding polycationic component. The targeting peptide may comprise a muscle cell targeting sequence, a linker, and a closed linear DNA-binding component (e.g. a closed linear DNA-binding polycationic component). The targeting peptide may comprise a muscle cell targeting sequence, a linker, and a linear DNA-binding component (e.g. a linear DNA-binding polycationic component).

The targeting peptide may comprise:
(a) a muscle cell targeting sequence;
(b) a linker
(c) a spacer; and
(d) a nucleic acid-binding component.

The targeting peptide may comprise a muscle cell targeting sequence, a linker, a spacer, and a nucleic acid-binding cationic component. The targeting peptide may comprise a muscle cell targeting sequence, a linker, a spacer, and a nucleic acid-binding polycationic component. The targeting peptide may comprise a muscle cell targeting sequence, a linker, a spacer, and a closed linear DNA-binding component (e.g. a closed linear DNA-binding polycationic component). The targeting peptide may comprise a muscle cell targeting sequence, a linker, a spacer, and a linear DNA-binding component (e.g. a linear DNA-binding polycationic component).

Preferably, the muscle cell targeting sequence is SEQ ID NO: 1, SEQ ID NO: 2, or SEQ ID NO: 3. Thus, the targeting peptide may comprise:
(a) a muscle cell targeting sequence, wherein the muscle cell targeting sequence is: SEQ ID NO: 1, SEQ ID NO: 2, or SEQ ID NO: 3; and
(b) a nucleic acid-binding component (e.g. a DNA-binding polycationic component).

The targeting peptide may comprise:
(a) a muscle cell targeting sequence, wherein the muscle cell targeting sequence is: SEQ ID NO: 1, SEQ ID NO: 2, or SEQ ID NO: 3;
(b) a spacer; and
(c) a nucleic acid-binding component (e.g. a DNA-binding polycationic component).

The targeting peptide may comprise:
(a) a muscle cell targeting sequence, wherein the muscle cell targeting sequence is: SEQ ID NO: 1, SEQ ID NO: 2, or SEQ ID NO: 3;
(b) a linker; and
(c) a nucleic acid-binding component (e.g. a DNA-binding polycationic component).

The targeting peptide may comprise:
(a) a muscle cell targeting sequence, wherein the muscle cell targeting sequence is: SEQ ID NO: 1, SEQ ID NO: 2, or SEQ ID NO: 3;
(b) a linker;
(c) a spacer; and
(d) a nucleic acid-binding component (e.g. a DNA-binding polycationic component).

The targeting peptide may comprise:
(a) a muscle cell targeting sequence, wherein the muscle cell targeting sequence is: SEQ ID NO: 1, SEQ ID NO: 2, or SEQ ID NO: 3; and
(b) a spacer.

The targeting peptide may comprise:
(a) a muscle cell targeting sequence, wherein the muscle cell targeting sequence is: SEQ ID NO: 1, SEQ ID NO: 2, or SEQ ID NO: 3; and
(b) a linker.

The targeting peptide may comprise:
(a) a muscle cell targeting sequence, wherein the muscle cell targeting sequence is: SEQ ID NO: 1, SEQ ID NO: 2, or SEQ ID NO: 3;
(b) a linker; and
(c) a spacer.

### 3. Nucleic acid cargos

The cargo suitable for use in the nanoparticle (e.g. non-viral transfection complex) may be any biomolecule or a small molecule. For example, the cargo may be a nucleic acid, a peptide, a polypeptide, or a protein. The cargo may be a fragment of a nucleic acid, a peptide, a polypeptide, or a protein.

The nucleic acid may be a DNA molecule or an RNA molecule. The DNA molecule may be a linear DNA molecule or circular DNA molecule. The nucleic acid may be single-stranded, double-stranded, or partially single-stranded and partially double-stranded.

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid comprises at least 500 nucleotides.

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid comprises at least 500 base pairs.

The circular DNA molecule may be a plasmid DNA, a vector DNA, a cosmid, an isolated DNA, a bacterial artificial chromosome, a minicircle, or a mini intronic plasmid (MIP). The circular DNA may be an enzymatically produced circular DNA molecule. For example, (i) a circular DNA molecule obtained from recombinase reaction (e.g. Cre recombinase reaction), or (ii) a circular DNA molecule obtained from ligase reaction (e.g. using the golden gate assembly).

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The closed linear DNA molecule has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open-ended cassettes have been demonstrated to be inefficient with respect to gene expression when introduced into host tissue. This has been attributed to cassette instability due to the action of exonucleases in the extracellular space.

Sequestering DNA ends inside closed structures also has other advantages. The DNA ends are prevented from integrating with genomic DNA and so closed linear DNA molecules offer improved safety. In addition, the closed linear structure reduces concatamerisation of DNA molecules inside host cells and thus expression levels of the gene product can be regulated in a more sensitive manner.

The closed linear DNA molecule may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The closed linear DNA molecule may comprise a spacer region. The spacer region may comprise at least 10, at least 20, at least 30, at least 40, at least 50, at least 60, at least 70, at least 80, at least 90, at least 100, at least 125, at least 150, at least 175, or at least 200 base pairs.

The closed linear DNA molecule may comprise an inverted terminal repeat sequence.

The closed linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the closed linear DNA molecule comprises at least 500 base pairs.

The closed linear DNA molecule may comprise one or more protected nucleotides (i.e. nucleotides resistant to nuclease (e.g. exonuclease) digestion). The closed linear DNA molecule may comprise one or more of protected nucleotides (i.e. nucleotides resistant to nuclease (e.g. exonuclease) in each strand. For example, the closed linear DNA molecule may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) in each strand.

The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The protected nucleotides may be located at the 3' and/or 5' end and/or 3' and/or 5' end region of the linear double-stranded DNA molecule. Preferably, the protected nucleotides are located at the 3' and 5' end and at the 3' and 5' region of the linear double-stranded DNA molecule. The 3' region comprises at least 20 3'-end nucleotides of the linear DNA molecule. The 3' region comprises less than 30 3'-end nucleotides of the linear DNA molecule. The 5' region comprises no more than 30 5'-end nucleotides of the linear DNA molecule. That is to say that the nucleotides which are the last and/or the first nucleotides in the linear DNA molecule may be protected nucleotides. In other words, the linear DNA molecule may comprise a "cap" of protected nucleotides, which protects the ends of the linear DNA molecule from nuclease (e.g. exonuclease) digestion.

The protected nucleotides (i.e. nucleotides resistant to nuclease (e.g. exonuclease) digestion) may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The protected nucleotides (i.e. nucleotides resistant to nuclease (e.g. exonuclease) digestion) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The linear double-stranded DNA molecule may comprise one or more protected nucleotides (e.g. phosphorothioated nucleotides) at internal positions in each strand (i.e. the sense and antisense strands).

The internal positions may be any positions in the linear double-stranded DNA molecule other than the last nucleotide on the 3'-end and the 5'-end of the sense and antisense strands. The internal positions may be located at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule. The internal positions may be located at least 7 nucleotides away from the 3'-end and/or the 5'-end of each strand of the linear double-stranded DNA molecule. Preferably, the internal positions are located at least 10 nucleotides away from the 3'-end and/or the 5'-end of each strand of the linear double-stranded DNA molecule. In the linear double-stranded DNA molecule, the internal positions may be located at least 7 nucleotides away from the 3'-end and the 5'-end of the sense strand. Preferably, in the linear double-stranded DNA molecule, the internal positions are located at least 10 nucleotides away from the 3'-end and the 5'-end of the sense strand. In the linear double-stranded DNA molecule, the internal positions may be located at least 7 nucleotides away from the 3'-end and the 5'-end of the antisense strand. Preferably, in the linear double-stranded DNA molecule, the internal positions may be located at least 10 nucleotides away from the 3'-end and the 5'-end of the antisense strand. In the linear double-stranded DNA molecule, the internal positions may be located at least 7 nucleotides away from the 3'-end of each strand. Preferably, in the linear double-stranded DNA molecule, the internal positions may be located at least 10 nucleotides away from the 3'-end of each strand. In the linear double-stranded DNA molecule, the internal positions may be located at least 7 nucleotides away from the 5'-end of each strand. Preferably, in the linear double-stranded DNA molecule, the internal positions may be located at least 10 nucleotides away from the 5'-end of each strand.

The linear double-stranded DNA molecule may comprise a cassette. The location of the protected (e.g. phosphorothioated) nucleotides in the linear double-stranded DNA molecule may be such that the cassette is protected from nuclease (e.g. exonuclease III) digestion. Thus, the one or more phosphorothioated nucleotides at the internal positions in each strand may be selected from:
(a) a 5'-end nucleotide of the cassette;
(b) a 3'-end nucleotide of the cassette; and
(c) one or more nucleotides outside of the cassette

The term *"5'-end nucleotide of the cassette"* as used herein is intended to encompass the 5'-end nucleotide of each strand of the cassette. Thus, in the linear double-stranded DNA molecule, the cassette typically comprises a 5'-end nucleotide of the sense strand and a 5'-end nucleotide of the antisense strand.

The term *"3'-end nucleotide of the cassette"* as used herein is intended to encompass the 3'-end nucleotide of each strand of the cassette. Thus, in the linear double-stranded DNA molecule, the cassette typically comprises a 3'-end nucleotide of the sense strand and a 3'-end nucleotide of the antisense strand.

The term *"outside of the cassette"* as used herein is intended to encompass any nucleotides which are not part of the cassette. This includes any nucleotides that are comprised in the linear double-stranded DNA molecule and which also do not form part of the cassette. The term *"N nucleotides outside of the cassette"* or *"N nucleotides away from the cassette"* is intended to describe nucleotides that are located N nucleotides from the end of the cassette towards the end of the linear double-stranded DNA molecule. For example, the term *"2 nucleotides outside of the cassette"* in the context of internal positions of nucleotides is meant to describe a nucleotide that is outside of the cassette and that is two nucleotides away from the last nucleotide of the cassette. For example, in the sequence: 5'-AAAAAACATAAAA (SEQ ID NO: 37), wherein the cassette starts from nucleotide "T" (in the 5' to 3' direction), the term *"2 nucleotides outside of the cassette"* refers to the "C" nucleotide. Thus, the term *"at least 2 nucleotides outside of the cassette"* or *"at least 2 nucleotides away from the cassette"* is meant to describe nucleotides that are outside of the cassette and that are at least two nucleotides away from the last nucleotide of the cassette. In the example above, nucleotides *"at least 2 nucleotides away from the cassette"* would be any nucleotides selected from 5'-AAAAAAC. Similarly, the term *"at least 2 nucleotides away from the 5'-end of the cassette"* is meant to describe nucleotides that are outside of the cassette and that are at least two nucleotides away from the last nucleotide at the 5'-end of the cassette. In the example above, the last nucleotide at the 5'-end of the cassette is "T", and nucleotides *"at least 2 nucleotides away from the 5'-end of the cassette"* would be any nucleotides selected from 5'-AAAAAAC.

The internal positions may not be located between the second and penultimate nucleotide of the cassette. The internal positions may be any position in the linear double-stranded DNA molecule other than the last nucleotide on the 3'-end and the 5'-end of the sense and antisense strands and other than nucleotides located between the second and penultimate nucleotide of the cassette. The internal positions may be located outside of the cassette and at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule and/or at least 2, 3, 4, 5, 6, 7, 8, 9, 10, 12, 14, 16, 18, 20, 30, 40, 50, 75, or 100 nucleotides away (i.e. outside of the cassette) from the 3'-end and/or the 5'-end of each strand of the cassette. Preferably, the internal positions is located outside of the cassette and at least 6, at least 8, or at least 10 nucleotides away from the 3'-end and/or the 5'-end of each strand of the linear double-stranded DNA molecule and/or at least 6, at least 8, or at least 10 nucleotides away from the 3'-end and/or the 5'-end of each strand of the cassette (i.e. at least 6, at least 8, or at least 10 nucleotides outside of the cassette). Preferably, the cassette does not comprise a phosphorothioated nucleotide at either one of the positions 1-2, 1-3, 1-4, 1-5, 1-6, 1-7, 1-8, 1-9, 1-10, 1-12, 1-14, 1-16, 1-18, or 1-20 of the sense and/or the antisense strand away from the 3'-end and/or the 5'-end of the cassette (i.e. outside of the cassette). Preferably, the internal positions in each strand are located at least 6 nucleotides away from the end of the linear double-stranded DNA molecule and are not located between the second and penultimate nucleotide of the cassette. Preferably, the internal positions in each strand are located at least 10 nucleotides away from the end of the linear double-stranded DNA molecule and are not located between the second and penultimate nucleotide of the cassette. Preferably, the internal positions in each strand are located at least 6 nucleotides away from the end of the linear double-stranded DNA molecule and at least 6 nucleotides away from the end of the cassette (i.e. at least 6 nucleotides outside of the cassette). Preferably, the internal positions in each strand are located at least 10 nucleotides away from the end of the linear double-stranded DNA molecule and at least 10 nucleotides away from the end of the cassette (i.e. at least 10 nucleotides outside of the cassette). The linear double-stranded DNA molecule may comprise a first phosphorothioated nucleotide at an internal position which is a position other than the last nucleotide on the 3'-end and the 5'-end of the sense and antisense strands as long as this position is located at least 2, 3, 4, 5, 6, 7, 8, 9, 10, 12, 14, 16, 18, 20, 30, 40, or 50 nucleotides outside of the cassette. For example, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are not located between the second and penultimate nucleotide of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located outside of the cassette. The linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 2, 3, 4, 5, 6, 7, 8, 9, 10, 12, 14, 16, 18, 20, 30, 40, or 50 nucleotides outside of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 6, 8, or 10 nucleotides outside of the cassette.

The linear double-stranded DNA molecule may comprise at least 2, 3, 4, 5, 6, 7, 8, 9 or 10 protected (e.g. phosphorothioated) nucleotides at internal positions in each strand. Preferably, the linear double-stranded DNA molecule comprises at least 2 protected (e.g. phosphorothioated) nucleotides at internal positions in each strand.

The location of phosphorothioated nucleotides in the sense strand of the linear double-stranded DNA molecule may be such that the one or more phosphorothioated nucleotides are located upstream of the cassette (i.e. towards the 5'-end of the sense strand of the DNA molecule).

In the sense strand of the linear double-stranded DNA molecule:
(a) one of the at least two phosphorothioated nucleotides may be the 5'-end nucleotide of the cassette; and/or
(b) one of the at least two phosphorothioated nucleotides may be in a first region of the sense strand, wherein the first region of the sense strand is 5' of the cassette.

The location of phosphorothioated nucleotides in the sense strand of the linear double-stranded DNA molecule may be such that the one or more phosphorothioated nucleotides are located downstream of the cassette (i.e. towards the 3'-end of the sense strand of the DNA molecule).

In the sense strand of the linear double-stranded DNA molecule:
(a) one of the at least two phosphorothioated nucleotides may be the 3'-end nucleotide of the cassette; and/or
(b) one of the at least two phosphorothioated nucleotides may be in a second region of the sense strand, wherein the second region of the sense strand is 3' of the cassette.

The location of phosphorothioated nucleotides in the antisense strand of the linear double-stranded DNA molecule may be such that the one or more phosphorothioated nucleotides are located upstream of the cassette (i.e. towards the 5'-end of the antisense strand of the DNA molecule).

In the antisense strand of the linear double-stranded DNA molecule:
(a) one of the at least two phosphorothioated nucleotides may be the 5'-end nucleotide of the cassette; and/or
(b) one of the at least two phosphorothioated nucleotides may be in a first region of the antisense strand, wherein the first region of the antisense strand is 5' of the cassette.

The location of phosphorothioated nucleotides in the antisense strand of the linear double-stranded DNA molecule may be such that the one or more phosphorothioated nucleotides are located downstream of the cassette (i.e. towards the 3'-end of the antisense strand of the DNA molecule).

In the antisense strand of the linear double-stranded DNA molecule:
(a) one of the at least two phosphorothioated nucleotides may be the 3'-end nucleotide of the cassette; and/or
(b) one of the at least two phosphorothioated nucleotides may be in a second region of the antisense strand, wherein the second region of the antisense strand is 3' of the cassette.

The location of phosphorothioated nucleotides in both the sense and the antisense strand of the linear double-stranded DNA molecule may be such that at least one phosphorothioated nucleotide is located downstream of the cassette and at least one phosphorothioated nucleotide is located upstream of the cassette in each strand.

In the linear double-stranded DNA molecule:
(a) in the sense strand one of the at least two phosphorothioated nucleotides may be the 5'-end nucleotide of the cassette, and/or one of the at least two phosphorothioated nucleotides may be in a first region of the sense strand, wherein the first region of the sense strand is 5' of the cassette;
(b) in the sense strand one of the at least two phosphorothioated nucleotides may be the 3'-end nucleotide of the cassette, and/or one of the at least two phosphorothioated nucleotides may be in a second region of the sense strand, wherein the second region of the sense strand is 3' of the cassette;
(c) in the antisense strand one of the at least two phosphorothioated nucleotides may be the 5'-end nucleotide of the cassette, and/or one of the at least two phosphorothioated nucleotides may be in a first region of the antisense strand, wherein the first region of the antisense strand is 5' of the cassette; and
(d) in the antisense strand one of the at least two phosphorothioated nucleotides may be the 3'-end nucleotide of the cassette, and/or one of the at least two phosphorothioated nucleotides may be in a second region of the antisense strand, wherein the second region of the antisense strand is 3' of the cassette.

The term *"first region of the sense strand"* as used herein is intended to encompass the part of the sense strand of the linear double-stranded DNA molecule that is between the 5'-end of the linear double-stranded DNA molecule and the first 5' nucleotide of the cassette in the sense strand. For example, in the sequence of the sense strand: 5'-AAAAAACATAAAA-3' (SEQ ID NO: 37), wherein the cassette starts from nucleotide "T" in the 5'-3' direction, the term *"first region of the sense strand"* refers to the 5'-AAAAAACA-3' region.

The term *"first region of the antisense strand"* is intended to encompass the part of the antisense strand of the linear double-stranded DNA molecule that is between the 5'-end of the linear double-stranded DNA molecule and the first 5' nucleotide of the cassette in the antisense strand. For example, in the sequence of the antisense strand: 5'-AAAAAACATAAAA-3' (SEQ ID NO: 37), wherein the cassette starts from nucleotide "T" in the 5'-3' direction, the term *"first region of the antisense strand"* refers to the 5'-AAAAAACA-3' region.

The term *"second region of the sense strand"* is intended to encompass the part of the sense strand of the linear double-stranded DNA molecule that is between the 3'-end of the linear double-stranded DNA molecule and the first 3' nucleotide of the cassette in the sense strand. For example, in the sequence of the sense strand: 5'-AAAAAACATAAAA-3' (SEQ ID NO: 37), wherein the cassette starts from nucleotide "T" in the 3'-5' direction, the term *"second region of the sense strand*" refers to the 5'-AAAA-3' region.

The term *"second region of the antisense strand"* is intended to encompass the part of the antisense strand of the linear double-stranded DNA molecule that is between the 3'-end of the linear double-stranded DNA molecule and the first 3' nucleotide of the cassette in the antisense strand. For example, in the sequence of the antisense strand: 5'-AAAAAACATAAAA-3' (SEQ ID NO: 37), wherein the cassette starts from nucleotide "T" in the 3'-5' direction, the term *"second region of the antisense strand"* refers to the 5'-AAAA-3' region.

The linear double-stranded DNA molecule may comprise a plurality of the phosphorothioated nucleotides upstream (i.e. towards the 5'-end of the sense strand of the DNA molecule) of the cassette. For example, the linear double-stranded DNA molecule may comprise at least 2, 3, 4, 5, 6, 7, 8, 9 or 10 phosphorothioated nucleotides upstream of the cassette. Preferably, at least 2 phosphorothioated nucleotides upstream of the cassette. Thus, the location of phosphorothioated nucleotides in the sense strand of the linear double-stranded DNA molecule may be such that at least two phosphorothioated nucleotides are located upstream of the cassette.

In the sense strand of the linear double-stranded DNA molecule:
(a) one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
(b) the at least two phosphorothioated nucleotides may be in a first region of the sense strand, wherein the first region of the sense strand is 5' of the cassette.

The linear double-stranded DNA molecule may comprise a plurality of the phosphorothioated nucleotides downstream (i.e. towards the 3-end of the DNA molecule) of the cassette. For example, the linear double-stranded DNA molecule may comprise at least 2, 3, 4, 5, 6, 7, 8, 9 or 10 phosphorothioated nucleotides downstream of the cassette, preferably at least 2 phosphorothioated nucleotides downstream of the cassette. Thus, the location of phosphorothioated nucleotides in the sense strand of the linear double-stranded DNA molecule may be such that at least two phosphorothioated nucleotides are located downstream of the cassette.

In the sense strand of the linear double-stranded DNA molecule:
(a) one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
(b) the at least two phosphorothioated nucleotides may be in a first region of the sense strand, wherein the first region of the sense strand is 3' of the cassette.

The location of phosphorothioated nucleotides in the antisense strand of the linear double-stranded DNA molecule may be such that at least two phosphorothioated nucleotides are located upstream of the cassette (i.e. towards the 5'-end of the antisense strand of the DNA molecule).

In the antisense strand of the linear double-stranded DNA molecule:
(a) one of the at least two phosphorothioated nucleotides is the 5'-end nucleotide of the cassette and one of the at least two phosphorothioated nucleotides is in a first region of the antisense strand, wherein the first region of the antisense strand is 5' of the cassette; or
(b) the at least two phosphorothioated nucleotides are in a first region of the antisense strand, wherein the first region of the antisense strand is 5' of the cassette.

The location of phosphorothioated nucleotides in the antisense strand of the linear double-stranded DNA molecule may be such that at least two phosphorothioated nucleotides are located downstream of the cassette (i.e. towards the 3'-end of the antisense strand of the DNA molecule).

In the antisense strand of the linear double-stranded DNA molecule:
(a) one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
(b) the least two phosphorothioated nucleotides may be in a second region of the antisense strand, wherein the second region of the antisense strand is 3' of the cassette.

Each strand of the linear double-stranded DNA molecule may comprise a plurality of phosphorothioated nucleotides. For example, the linear double-stranded DNA molecule may comprise at least 2, 3, 4, 5, 6, 7, 8, 9 or 10 phosphorothioated nucleotides upstream and downstream of the cassette. The location of phosphorothioated nucleotides in both the sense and the antisense strand of the linear double-stranded DNA molecule may be such that the at least two phosphorothioated nucleotides are located downstream of the cassette and at least two phosphorothioated nucleotides are located upstream of the cassette in each strand.

In the linear double-stranded DNA molecule:
(a) in the sense strand:
   i. one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
   ii. the at least two phosphorothioated nucleotides may be in a first region of the sense strand, wherein the first region of the sense strand is 5' of the cassette;
(b) in the sense strand:
   i. one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
   ii. the at least two phosphorothioated nucleotides may be in a second region of the sense strand, wherein the second region of the sense strand is 3' of the cassette;
(c) in the antisense strand:
   i. one of the at least two phosphorothioated nucleotides may be the 5'-end nucleotide of the cassette and one of the at least two phosphorothioated nucleotides may be in a first region of the antisense strand, wherein the first region of the antisense strand is 5' of the cassette; or
   ii. the at least two phosphorothioated nucleotides may be in a first region of the antisense strand, wherein the first region of the antisense strand is 5' of the cassette; and
(d) in the antisense strand:
   i. one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
   ii. the at least two phosphorothioated nucleotides may be in a second region of the antisense strand, wherein the second region of the antisense strand is 3' of the cassette.

The linear double-stranded DNA molecule or the closed linear DNA molecule may be resistant to nuclease digestion or may have improved or enhanced resistance to nuclease digestion. The linear double-stranded DNA molecule or the closed linear DNA molecule may be resistant to exonuclease digestion or have improved or enhanced resistance to exonuclease digestion. The linear double-stranded DNA molecule or the closed linear DNA molecule may be resistant, or have improved or enhanced resistance, to digestion by exonucleases that cleave the 3'-end nucleotides (e.g. exonuclease III) and/or exonucleases that cleave the 5'-end nucleotides (e.g. exonuclease VIII). The terms "improved" or "enhanced" in the context of resistance to enzyme digestion refer to higher resistance to enzyme digestion when compared to a DNA molecule not produced by the methods described herein. For example, for the linear double-stranded DNA molecule, when compared to a molecule that does not comprise protected nucleotides, such as phosphorothioated nucleotides.

### 4. Pharmaceutical compositions

The invention provides a pharmaceutical composition comprising a nanoparticle (e.g. a non-viral transfection complex) described herein and a pharmaceutically suitable carrier or excipient. The invention provides a pharmaceutical composition comprising a targeting peptide described herein and a pharmaceutically suitable carrier or excipient.

The invention provides a pharmaceutical composition comprising:
i. a nanoparticle (e.g. a non-viral transfection complex), which comprises:
   (a) a cargo;
   (b) a lipid component; and
   (c) a targeting peptide comprising a muscle cell targeting sequence; and
ii. a pharmaceutically suitable carrier.

The pharmaceutical composition may comprise:
i. a nanoparticle (e.g. a non-viral transfection complex), which comprises:
   (a) a cargo
   (b) a lipid component; and
   (c) a targeting peptide comprising a muscle cell targeting sequence and a cargo-binding component; and
ii. a pharmaceutically suitable carrier.

The pharmaceutical composition may comprise:
i. a nanoparticle (e.g. the non-viral transfection complex), which comprises:
   (a) a cargo, wherein the cargo is a nucleic acid;
   (b) a lipid component; and
   (c) a targeting peptide comprising a muscle cell targeting sequence and a nucleic acid-binding polycationic component (e.g. DNA-binding polycationic component); and
ii. a pharmaceutically suitable carrier.

The pharmaceutical composition may comprise:
i. a nanoparticle (e.g. the non-viral transfection complex), which comprises:
   (a) a cargo, wherein the cargo is a nucleic acid, wherein the nucleic acid is a closed linear DNA molecule;
   (b) a lipid component; and
   (c) a targeting peptide comprising a muscle cell targeting sequence and a closed linear DNA-binding component (e.g. a closed linear DNA-binding polycationic component); and
ii. a pharmaceutically suitable carrier.

The pharmaceutical composition may comprise:
i. a nanoparticle (e.g. the non-viral transfection complex) may comprise:
   (a) a cargo, wherein the cargo is a nucleic acid, wherein the nucleic acid is a linear DNA molecule comprising one or more nuclease-resistant nucleotides (e.g. phosphorothioated nucleotides);
   (b) a lipid component; and
   (c) a targeting peptide comprising a muscle cell targeting sequence and a linear DNA-binding component (e.g. a linear DNA-binding polycationic component); and
ii. a pharmaceutically suitable carrier.

The pharmaceutical composition may comprise:
i. a targeting peptide comprising:
   (a) a muscle cell targeting sequence; and
   (b) a cargo-binding component; and
ii. a pharmaceutically suitable carrier.

The pharmaceutical composition may comprise:
i. a targeting peptide comprising:
   (a) a muscle cell targeting sequence
   (b) a spacer; and
   (c) a cargo-binding component; and
ii. a pharmaceutically suitable carrier.

The pharmaceutical composition may comprise:
i. a targeting peptide comprising:
   (a) a muscle cell targeting sequence;
   (b) a linker; and
   (c) a cargo-binding component; and
ii. a pharmaceutically suitable carrier.

The pharmaceutical composition may comprise:
i. a targeting peptide comprising:
   (a) a muscle cell targeting sequence;
   (b) a linker;
   (c) a spacer; and
   (d) a cargo-binding component; and
ii. a pharmaceutically suitable carrier.

The muscle cell targeting sequence may be any of the muscle cell targeting sequences described herein. Preferably, the muscle cell targeting sequence is SEQ ID NO: 1, SEQ ID NO: 2, or SEQ ID NO: 3, or a variant thereof comprising one or more conservative amino acid substitutions.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally, transdermally, intramuscularly, or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

The pharmaceutical composition may be administered by injection (e.g. intravenous or intramuscular injection). The pharmaceutical composition may be formulated as an emulsion consisting of a sterile, pyrogen-free preparation or a lipid complex preparation.

The pharmaceutical composition may be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

In another mode of administration, the pharmaceutical composition can be administered in situ, via a catheter or pump. A catheter or pump can, for example, direct the composition into the target location.

The parenteral carrier system (e.g. intravenous carrier system or intramuscular carrier system) may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 5. Uses and applications

### General therapeutic and diagnostic uses

The invention provides the nanoparticle described herein for use in therapy.

The invention provides the targeting peptide described herein for use in therapy.

The invention also provides the pharmaceutical composition described herein for use in therapy.

The nanoparticle may comprise a cargo (e.g. a nucleic acid cargo) which encodes a sequence of a therapeutic protein, a part of a vaccine, or an element of a genetic engineering mechanism, which is used to treat a disease or infection in a subject.

The nanoparticle described herein, the targeting peptide described herein, or the pharmaceutical composition described herein may be used in the treatment of a muscle disease or disorder. The muscle disease or disorder may be a genetic muscle disease or disorder. The genetic muscle disease may be muscular dystrophy e.g. Duchenne muscular dystrophy, myotonic dystrophy, facioscapulohumeral muscular dystrophy or Becker muscular dystrophy.

The nanoparticle described herein, the targeting peptide described herein, or the pharmaceutical composition described herein may be used as a medicament. The invention provides the nanoparticle described herein, the targeting peptide described herein, or the pharmaceutical composition described herein for use in the manufacture of a medicament for the prophylaxis of a condition caused in a subject by a defect and/or deficiency in a gene.

The invention provides a method for treatment of a disease or disorder caused in a subject by defect and/or deficiency in a gene, the method comprising administering the nanoparticle described herein, the targeting peptide described herein, or the pharmaceutical composition described herein to the subject. Preferably, the amount of the nanoparticle, the targeting peptide or the pharmaceutical composition administered to the subject is a therapeutically active amount. Thus, the invention also provides the nanoparticle described herein, the targeting peptide described herein, or the pharmaceutical composition described herein may be used in gene therapy.

A subject treated with the nanoparticle described herein, the targeting peptide described herein, or the pharmaceutical composition described herein may receive the nanoparticle described herein, the targeting peptide described herein, or the pharmaceutical composition described herein with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the nanoparticle described herein, the targeting peptide described herein, or the pharmaceutical composition described herein into the subject's body as described in more detail above (see "Pharmaceutical compositions"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the nanoparticle described herein, the targeting peptide described herein, or the pharmaceutical composition described herein that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides use of the nanoparticle described herein, the targeting peptide described herein, or the pharmaceutical composition described herein in a method of diagnosing a disease and/or a disorder.

The invention provides the use of the nanoparticle described herein, the targeting peptide described herein, or the pharmaceutical composition described herein in the "in vitro" diagnosis of a disease.

The invention also provides the nanoparticle described herein, the targeting peptide described herein, or the pharmaceutical composition described herein for use in a method of diagnosis "in vivo" of disease.

The method may be used to diagnose a muscle disease or disorder. The muscle disease or disorder may be a genetic muscle disease or disorder. The genetic muscle disease may be muscular dystrophy e.g. Duchenne muscular dystrophy, myotonic dystrophy, facioscapulohumeral muscular dystrophy or Becker muscular dystrophy.

The diagnostic and treatment methods described herein may be in vitro methods or in vivo methods.

The method of diagnosis may rely on the detection and/or quantification of the nanoparticle described herein, the targeting peptide described herein, or the pharmaceutical composition described herein.

To facilitate detection and/or quantification of the nanoparticle described herein, the nanoparticle may be attached or bound to a functional portion. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the nanoparticle For example, the signal may be measured by flow cytometry and/or fluorescence-activated cell sorting of the nanoparticle attached to a fluorescent probe.

The nanoparticle may be detected by being bound to a capture moiety, for example in a lateral flow assay. In this example, the functional portion is a protein, for example, an antibody specific for the capture moiety. The capture of the antibody attached to the nanoparticle may produce a visual signal (e.g. a band of a different colour).

The invention also provides a method that combines diagnosis of a disease with a treatment of the disease.

### Cell therapy

The nanoparticle described herein, the targeting peptide described herein, or the pharmaceutical composition described herein may be used in cell therapy.

The invention provides the use of the nanoparticle described herein, the targeting peptide described herein, or the pharmaceutical composition described herein in the production of cell therapy. The invention provides a method of cell therapy, comprising contacting the nanoparticle described herein, the targeting peptide described herein, or the pharmaceutical composition described herein with a cell. Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

### Vaccines

The products of the invention are particularly suitable for use in vaccine production. The nanoparticle described herein, the targeting peptide described herein, or the pharmaceutical composition described herein may be used to produce a vaccine, preferably an mRNA-based vaccine. For example, vaccines such as the BioNTech and Moderna mRNA vaccines against COVID-19.

The nanoparticle described herein or the pharmaceutical composition described herein may be used as a vaccine.

The nanoparticle described herein, the targeting peptide described herein, or the pharmaceutical composition described herein may be used in the therapeutic or prophylactic immunisation.

### CRISPR delivery

The nanoparticles and targeting peptides are particularly suitable for use in delivery of CRISPR machinery to cells, for example in cell therapy or in vivo therapy.

The nanoparticle may comprise a cargo, which is a nucleic acid cargo (e.g. the nucleic acid cargo described herein). The nucleic acid cargo may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template may comprise or consist of a homology region for a dystrophin gene or a part of the dystrophin gene. The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). For example, the repair template may introduce a correct (i.e. functional) copy of the dystrophin gene (i.e. the DMD gene) or a portion thereof. The repair template may comprise a correct (i.e. functional) copy of the dystrophin gene (i.e. the DMD gene) or a portion thereof. The repair template (or editing template) may comprise at least 10, at least 20, at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500, at least 8000, at least 8500, at least 9000, at least 9500, at least 10000, at least 11000, at least 12000, at least 13000, at least 14000, or at least 15000 base pairs.

Thus, the invention provides the nanoparticle for use in CRISPR system delivery to a cell. The invention provides a method of delivering the CRISPR system to a cell, comprising contacting the nanoparticle with a cell. Preferably, the cell is a muscle cell.

The cell may be an animal cell, preferably mammal cell, such as human cell.

The invention also provides a cell obtainable by the methods described herein. The cell is particularly suitable for use in cell therapy and/or in vivo therapy.

Uses of the products described herein may be *in vivo* or *in vitro* uses.

### 6. Methods for producing nanoparticles

The invention provides a method for producing (or forming) a nanoparticle described herein, wherein the method comprises:
(a) contacting a lipid component with a cargo and a targeting peptide comprising a muscle cell targeting sequence to form a single contiguous aqueous volume; and
(b) forming the nanoparticle.

The invention provides a method for producing (or forming) a nanoparticle described herein, wherein the method comprises:
(a) contacting a lipid component with a cargo and a targeting peptide comprising a muscle cell targeting sequence; and
(b) forming the nanoparticle.

The lipid component may be a liposome. The method may comprise the steps:
(a) contacting a liposome with a cargo and a targeting peptide comprising a muscle cell targeting sequence; and
(b) forming the nanoparticle.

The method may further comprise a step of forming a liposome. The step of forming a liposome may be performed before the step of contacting a liposome with a cargo and a targeting peptide comprising a muscle cell targeting sequence.

The step of forming the nanoparticle may be performed by shaking, pipetting or using a microfluidics device.

The method may further comprise a step of diluting the nanoparticle to a desired cargo concentration. The step of diluting the nanoparticle to a desired cargo concentration after the step of forming the nanoparticle. For example, if the cargo is a nucleic acid (e.g. DNA), the desired cargo concentration may be 0.01 ng/µL - 10,000 ng/µL, 0.1 ng/µL - 1000 ng/µL or 1 ng/µL - 100 ng/µL.

### 7. Cell transfection

The invention provides a cell transfection composition comprising the nanoparticle described herein.

The invention provides a method for transfecting a cell comprising:
(a) contacting a cell with the nanoparticle described herein; and
(b) transfecting the nanoparticle to the cell.

The cell may be an animal cell, such as mammal cell (e.g. a human cell), a fungal cell, a cell of a micro-organism (e.g. a prokaryotic cell or a eukaryotic cell), or a plant cell. The cell may be a human cell. The cell may be a C2C12 cell or a HEK293 cell.

The cell transfection composition may or may not comprise an agent selected from a photosensitizing agent and/or a radical initiator e.g. a photoinitiator. Preferably, this agent improves the function of the cargo (e.g. the nucleic acid cargo described herein) at a target site and/or protects the cargo (e.g. the nucleic acid cargo described herein) from metabolism and/or degradation.

The invention further provides a cell obtainable by the methods of the invention. Thus, the cell may comprise the nanoparticle of the invention.

The invention further provides a cell transfected with the nanoparticle described herein.

The step of contacting the cell with the nanoparticle may be performed in vivo. For example, the nanoparticle may be administered to an organism (e.g. a subject) in need thereof. The organism (e.g. the subject) may be an animal, such as mammal (e.g. a human), a fungus, a micro-organism, or a plant.

### BRIEF DESCRIPTION OF THE DRAWINGS

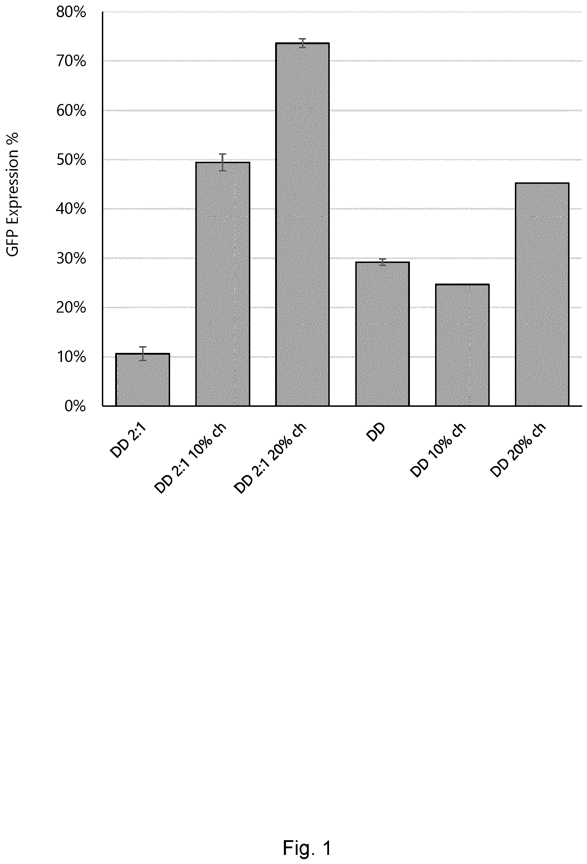

Figure 1 shows GFP expression and median fluorescence intensity of C2C12 cells transfected with DNA-lipid-peptide nanoparticles containing different molar percentages of cholesterol and a single muscle cell targeting peptide, MD1C. GFP expression was measured 48h post transfection. n=3 in all experiments, error bars = S.D. "DD" stands for an equimolar ratio of DOTMA:DOPE, while "DD 2:1" stands for a 2:1 molar ratio of DOTMA:DOPE. "Ch" is an abbreviation of cholesterol.

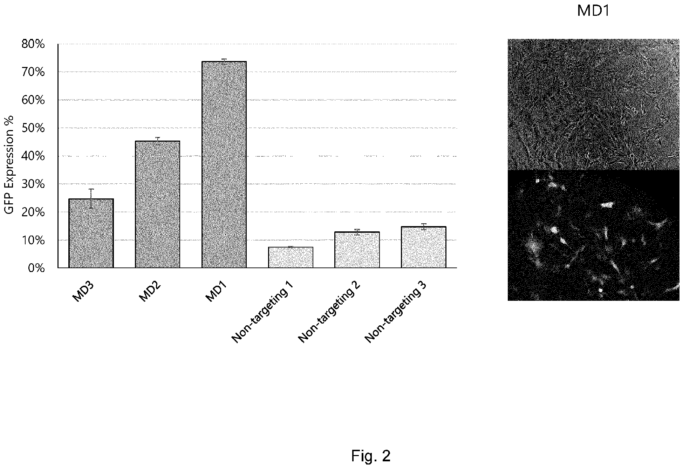

Figure 2 shows GFP expression in C2C12 cells transfected with plasmid DNA-lipid-peptide nanoparticles containing peptides with different targeting sequences specific for muscle cells. Non-targeting peptides were used as a control. GFP expression was measured 48h post transfection. n-=3 in all experiments, error bars = S.D.

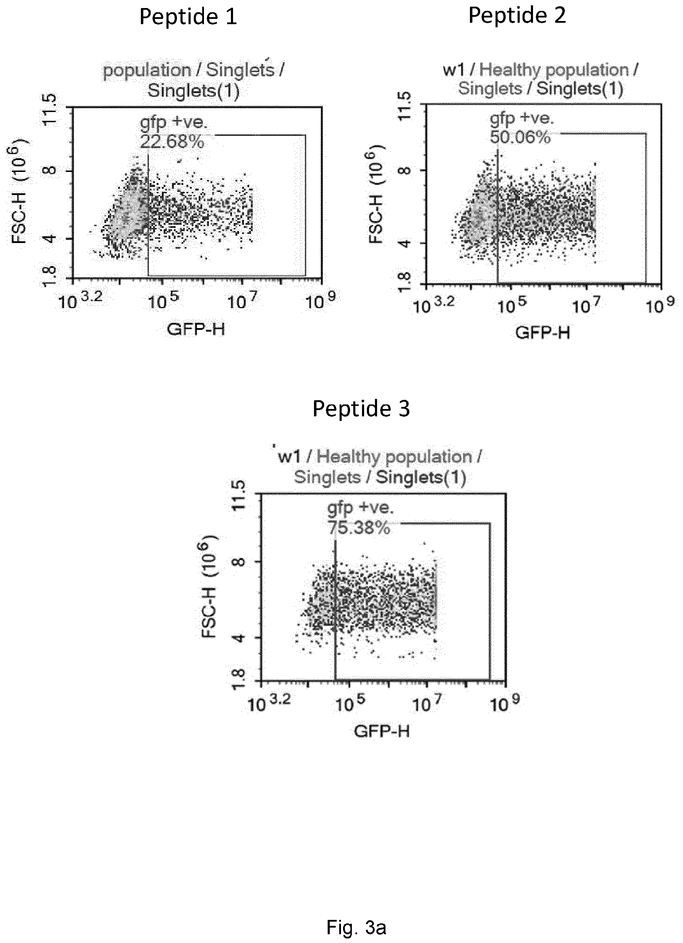

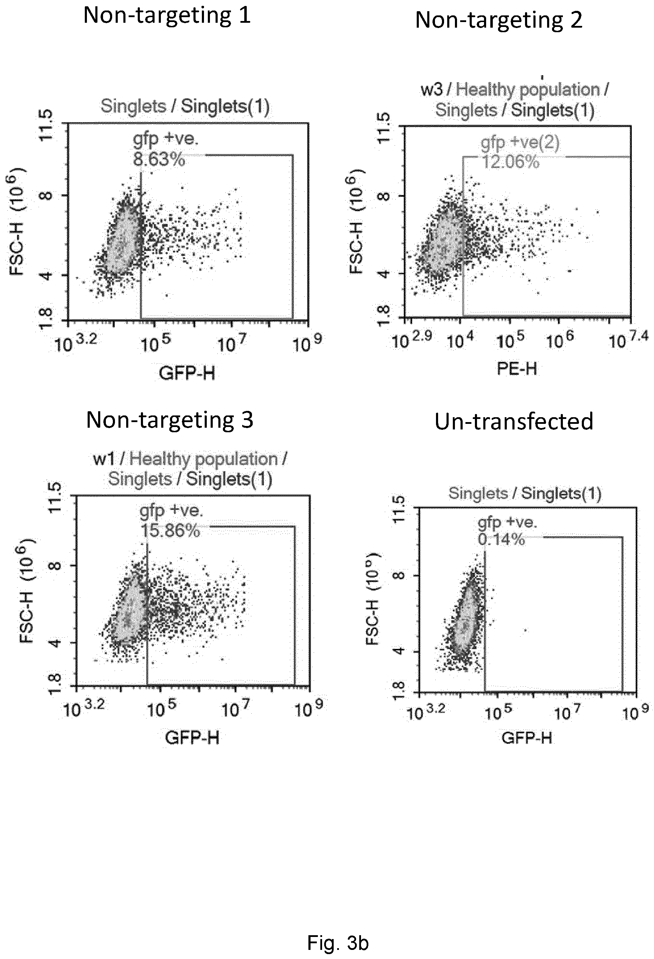

Figure 3 (3a and 3b) shows representative flow cytometry dot plots of GFP expression in C2C12 cells transfected with plasmid DNA-lipid-peptide nanoparticles containing peptides with different targeting ligands specific for muscle cells, as well as non-targeting controls. GFP expression was measured 48h post transfection. n=3 in all experiments, error bars = S.D.

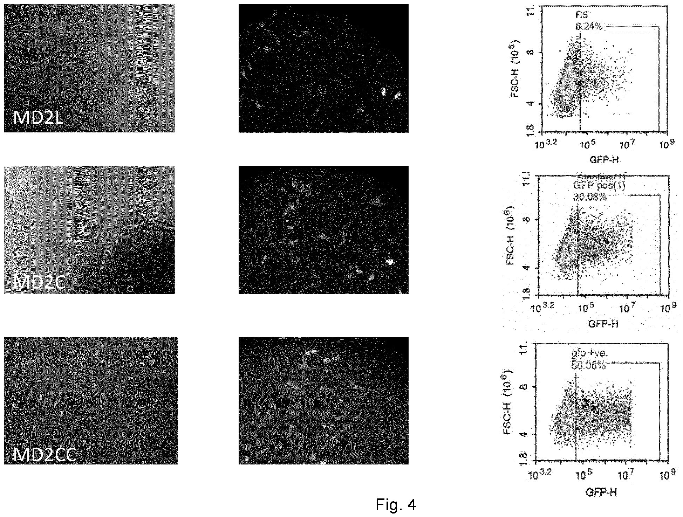

Figure 4 shows representative flow cytometry dot plots and microscopy images of GFP expression in C2C12 cells transfected with plasmid DNA-lipid-peptide nanoparticles containing a single targeting sequence specific for muscle cells with different structural conformations of targeting peptide. GFP expression was measured 48h post transfection. n=3 in all experiments, error bars = S.D.

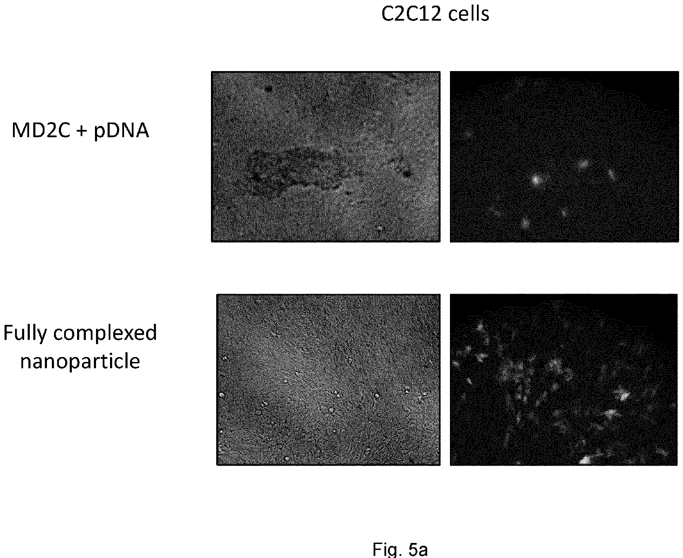

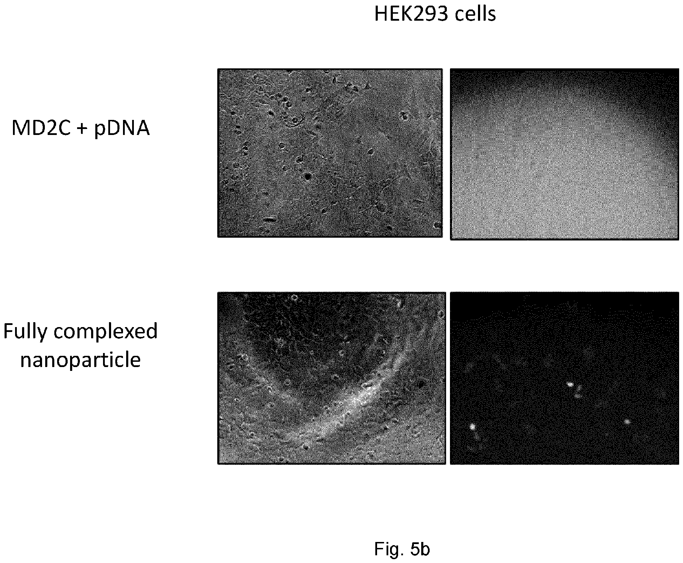

Figure 5 (5a and 5b) shows representative microscopy images of GFP expression in C2C12 and HEK293 cells transfected with plasmid DNA-peptide complexes, and DNA-lipid-peptide nanoparticles containing a single targeting peptide specific for muscle cells, MD2C. GFP expression was imaged 48h post transfection. n=3 in all experiments, error bars = S.D.

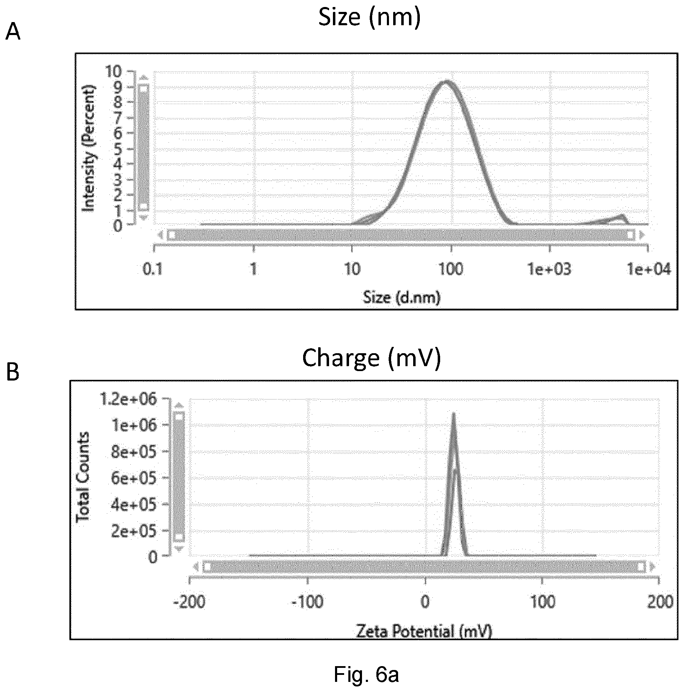

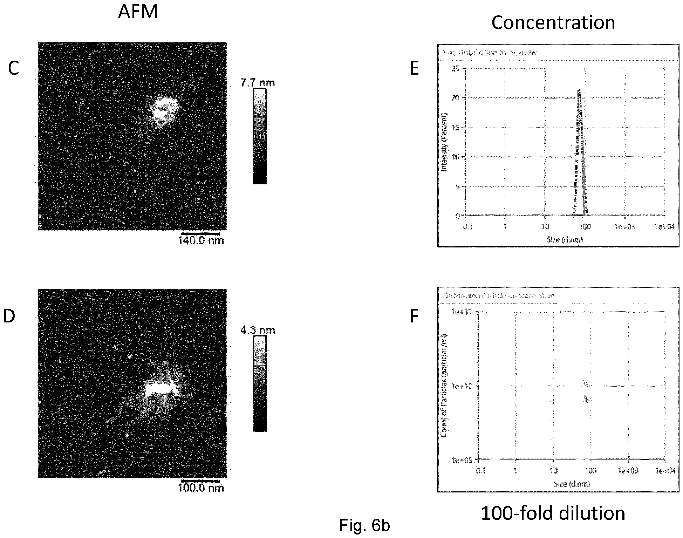

Figure 6 (Figure 6a and 6b) shows the biophysical characterisation of a representative plasmid DNA-lipid-peptide nanoparticle containing C18(DOTMA) DOPE 10% cholesterol and targeting ligand MD2C. A shows size of the particles in nm by Dynamic Light Scattering (DLS). B shows the zeta potential of the nanoparticle in mV Electrophoretic Light Scattering (ELS). C and D show representative atomic force microscopy images of nanoparticles. E and F show the concentration of the nanoparticles by Multi-Angle Dynamic Light Scattering (MADLS).

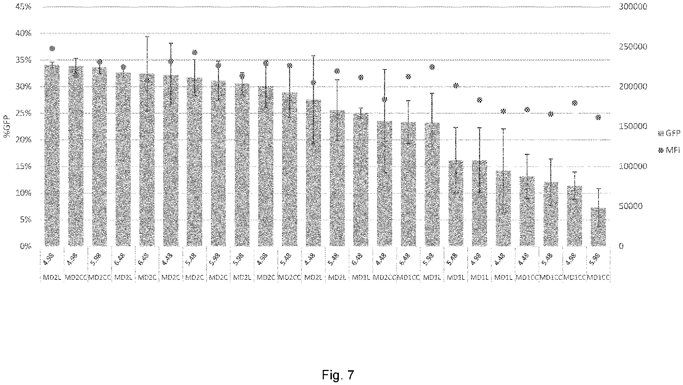

Figure 7 shows GFP expression in C2C12 cells transfected with closed linear DNA-lipid-peptide nanoparticles containing peptides with different targeting sequences specific for muscle cells, with different structural conformations of the targeting peptide. A range of lipids with varying molar ratios of DOTMA, DOPE and cholesterol were explored. GFP expression was measured 48h post transfection.
n=3 in all experiments, error bars = S.D.

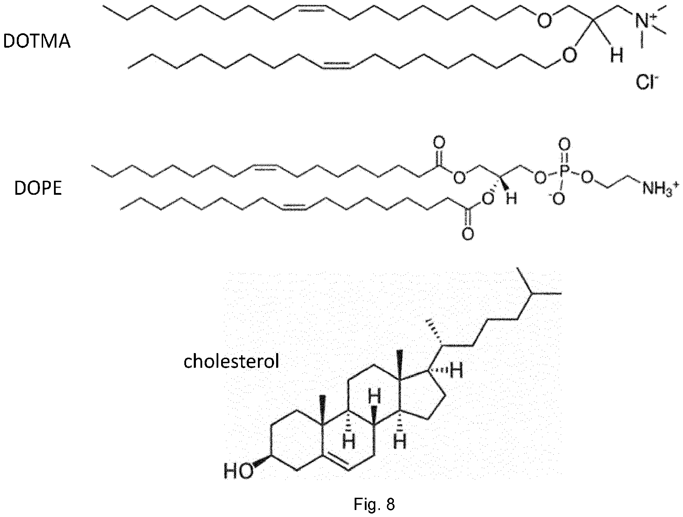

Figure 8 shows chemical structure of DOTMA, DOPE and cholesterol.

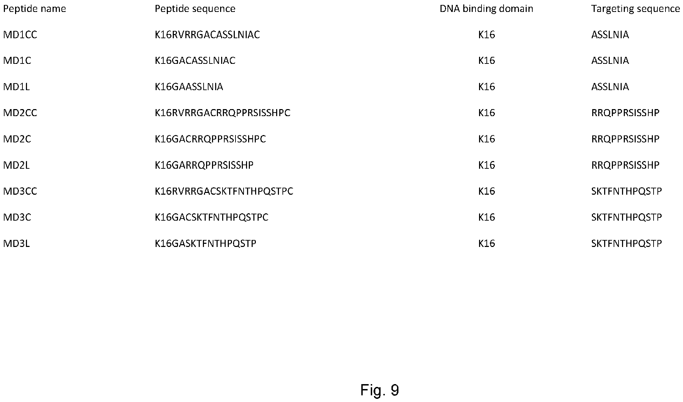

Figure 9 provides details of exemplary targeting peptides.

### EXAMPLES

### Example 1 - Liposome formulation

Liposomes were made using NanoAssemblr Ignite, a microfluidcs device (Precison Nanosystems, Vancouver Canada). The cationic lipid and phospholipid/cholesterol were mixed together at various molar ratios in ethanol and injected into the cartridge at a flow rate of 12mL/ min. They were then sonicated in a water bath for 20 minutes. The resulting liposomes were dialysed overnight in 10k Slide-A-Lyzer (Thermo Fisher) cassettes to remove any residual ethanol. Prior to use in experiments, liposomes were stored at 4°C.

### Example 2

Peptides identified in the literature with desirable muscle-cell binding characteristics (sequences are shown in Table 1) were synthesised via solid-phase peptide synthetic chemistry in various conformations (Gao et al. ("Effective dystrophin restoration by a novel muscle-homing peptide-morpholino conjugate in dystrophin-deficient mdx mice", Molecular Therapy, 22.7 (2014): 1333-1341); Yu et al. ("A muscle-targeting peptide displayed on AAV2 improves muscle tropism on systemic delivery", Gene therapy, 16.8 (2009): 953-962); and Seow and Wood. ("Identification of a novel muscle targeting peptide in mdx mice", Peptides, 31.10 (2010): 1873-1877)) . A sixteen-lysine tail group was attached using standard synthesis methods (amsbio, UK). Sequences are shown in Figure 9.

C2C12 cells, an immortalised mouse myoblast line, were cultured in Dulbecco's Modified Eagle's Medium (DMEM, Gibco) containing 10% fetal bovine serum (FBS, Gibco) and 1% pen/strep (Gibco).

Transfections in C2C12 cells were performed with a range of peptide: liposome: nucleic acid cargo ratios. Transfections were performed in a 96-well plate at a density of 8,000 cells per well seeded 24 h previously. Charge and mass ratio of components in the transfection complex varied; DNA was maintained at a constant mass of 300 ng per well, while lipid component was altered. In one case, the molar ratio of the cationic lipid C18 to the phospholipid DOPE was kept at 1:1 (DD). In another case, the molar ratio of the cationic lipid C18 to the phospholipid DOPE was kept at 2:1 (DD 2:1), while cholesterol was added at a molar percentage of 10 or 20% of the total lipid content. The mass ratio of linear DNA:liposome:peptide was maintained at 1:3:3, while the muscle-cell binding peptides were in some cases altered.

Transfection complexes were formed via electrostatic interaction by vigorous pipetting, before diluting in a final volume of 200 µl per well, with a DNA concentration of 1.5ng/µl . All dilutions were performed with OptiMEM reduced serum media. Controls included cells with no transfection complexes added (OptiMEM only).

Transfection complexes were incubated for 30 min at RT before adding to cells. All conditions were performed in triplicate. Cells were incubated at 37°C for 4 hours, before replacing media with growth media. 48 h post-transfection, the cells were rinsed in PBS, before incubation in 0.05% Trypsin EDTA (Gibco) to detach the cells. Cells were then re-suspended in PBS for analysis by flow cytometry.

In the following examples, the lipids used in the liposomes for nanoparticle formulation are denoted using the following abbreviations: C18 (DOTMA- 18:1(11c)Diether TAP- 1,2-di-O-octadecenyl-3-trimethylammonium propane). DD is an equimolar ratio of DOTMA:DOPE, while DD 2:1 is a 2:1 molar ratio of DOTMA:DOPE. Ch is an abbreviation of cholesterol.

Figure 1 shows GFP expression in C2C12 cells transfected with pDNA:lipid:peptide nanoparticles comprising different mass ratios of lipid and peptide components. DD 2:1 with 20% cholesterol + peptide MD1C showed highest GFP expression, with 73% of cells expressing GFP and a median fluorescent intensity (MFI) of 2.96E+06, indicating greatest transfection efficiency of these nanoparticles. DD 2:1 with 10%, following by DD 20% cholesterol + peptide MD1C showed the next highest GFP expression, with 49.5 and 45.2% of cells expressing GFP, with an MFI of 1.04E+06 and 9.54E+06. The lowest GFP expression was observed in HEK293 cells transfected with DD 2:1 with 0% cholesterol and peptide MD1C, with only 10.6% of cells expressing GFP.

Figure 2 shows GFP expression in C2C12 cells transfected with pDNA:lipid:peptide nanoparticles comprising DD 2:1 with 20% cholesterol with peptide sequences each identified to bind to muscle-cells. Nanoparticles containing non-targeting peptides were used as a control. Nanoparticles containing peptide MD1 showed highest GFP expression, with 73% of cells expressing GFP, indicating greatest transfection efficiency of these nanoparticles. MD2 containing particles lead to a GFP expression level of 50.1%. MD3 containing particles achieved the lowest transfection efficiency of 22.68%. The non-targeting peptides achieved a GFP expression efficiency ranging from 8.63% to 15.86%. A representative microscopy image is shown of HEK293 cells transfected with nanoparticles containing peptide MD1L. Representative flow cytometry dot plots are shown in Figure 3a (Peptide 1, 2 and 3 are MD1, MD2 and MD3, respectively) and Figure 3b (Non-targeting peptides 1, 2 and 3, and un-transfected cells).

Figure 4 shows representative microscopy images of C2C12 cells transfected with pDNA:lipid:peptide nanoparticles comprising DD 2:1 with 20% cholesterol with peptide sequence MD2 with different conformations of the targeting motif. MD1L has a linear targeting sequence, MD2C has a cyclic targeting sequence, while MD2CC has a cyclic targeting sequences preceded by a cleavable linker. MD2L containing particles achieved a GFP transfection efficiency of 8.24%, MD2C 30.08% and MD2CC 50.06%, indicating that the cyclic cleavable conformation of MD2 achieves the highest transfection efficiency in C2C12 cells.

Figure 5 shows the specificity of nanoparticles comprising peptide MD2C towards C2C12 cells, as compared to HEK293 cells. In the first panel, particles are formed using DNA and peptide only, and some GFP expression is visualised my microscopy in C2C12 cells, suggesting uptake of the particles. NO GFP expression can be seen in HEK293 cells. When the nanoparticle is fully formed with lipid components included, in this instance DD with 10% cholesterol, GFP expression is much higher in C2C12 cells as compared to HEK293 cells. The high expression in C2C12 compared with HEK293 cells is particularly impressive owing to the high transfection efficiency achievable in HEK293 cells.

Figure 7 shows GFP expression in C2C12 cells transfected with covalently-closed linear DNA-lipid-peptide nanoparticles containing peptides with different targeting ligands specific for muscle cells, with different structural conformations of the targeting ligand. A range of lipids with varying molar ratios of DOTMA, DOPE and cholesterol were explored. GFP expression was measured 48h post transfection. n=3 in all experiments, error bars = S.D.

### Example 3 - Biophysical characterisation of plasmid DNA nanoparticles

The size in nm by Dynamic Light Scattering (DLS), zeta potential in mV by Electrophoretic Light Scattering (ELS), and concentration of particles per ml by Multi-Angle Dynamic Light Scattering (MADLS) of the hpDNA-lipid-peptide nanoparticles was determined using the Zetasizer Ultra (Malvern). Complexes were prepared as before, except formulated in nuclease-free water rather than Opti-MEM. Samples containing 0.7 µg plasmid were diluted in 1 ml for analysis. The results show that the hpDNA nanoparticles have favourable biophysical characteristics.

Figure 6

## Claims

1. A nanoparticle comprising:
(a) a cargo;
(b) a lipid component; and
(c) a targeting peptide comprising a muscle cell targeting sequence.

2. The nanoparticle of claim 1, wherein the nanoparticle is a non-viral transfection complex.

3. The nanoparticle of claim 1 or claim 2, wherein the cargo is a biomolecule, optionally wherein the biomolecule is a nucleic acid, a peptide, a polypeptide, or a protein.

4. The nanoparticle of any one of claims 1-3, wherein the nanoparticle further comprises a cargo-binding component, optionally wherein the cargo-binding component is a cargo-binding cationic component, a cargo-binding neutral component or a cargo-binding anionic component.

5. The nanoparticle of any one of claims 1-4, wherein the cargo is a nucleic acid and wherein the targeting peptide comprises a nucleic acid-binding cationic component.

6. The nanoparticle of any one of claims 1-5, wherein the muscle cell targeting sequence comprises:
(a) wherein the targeting sequence is SEQ ID NO: 1 or a variant thereof comprising one or more conservative amino acid substitutions;
(b) wherein the targeting sequence is SEQ ID NO: 2 or a variant thereof comprising one or more conservative amino acid substitutions; or
(c) wherein the targeting sequence is SEQ ID NO: 3 or a variant thereof comprising one or more conservative amino acid substitutions.

7. The nanoparticle of any one of claims 1-5, wherein the targeting peptide comprises the sequence of any one of SEQ ID NO: 4-33, or a variant thereof comprising one or more conservative amino acid substitutions.

8. The nanoparticle of any one of the claims 1-7, wherein the nanoparticle is a non-viral transfection complex and wherein the non-viral transfection complex further comprises a cargo-binding component, wherein the cargo-binding component is a nucleic acid-binding component, optionally wherein the nucleic acid-binding component is a nucleic acid-binding cationic component.

9. A targeting peptide comprising:
(a) a muscle cell targeting sequence; and
(b) a cargo-binding component.

10. The targeting peptide of claim 9, wherein the targeting peptide further comprises a linker, optionally wherein the linker is cleavable or non-cleavable.

11. A targeting peptide comprising the sequence of any one of SEQ ID NO: 4-33, or a variant thereof comprising one or more conservative amino acid substitutions.

12. A pharmaceutical composition comprising a nanoparticle of anyone of claims 1-8, or the targeting peptide of any one of claims 9-11, and a pharmaceutically suitable carrier.

13. The nanoparticle of any one of claims 1-8, the targeting peptide of any one of claims 9-11, or the pharmaceutical composition of claim 12, for use in therapy.

14. The nanoparticle of any one of claims 1-8, the targeting peptide of any one of claims 9-11, or the pharmaceutical composition of claim 12, for use in the treatment of a muscle disease or disorder.

15. A method for transfecting a cell comprising:
(a) contacting a cell with the nanoparticle of any one of claims 1-8; and
(b) transfecting the nanoparticle to the cell.
